# EUROPÄISCHE PATENTSCHRIFT

(11) **EP 3 060 548 B1**
(45) Veröffentlichungstag und Bekanntmachung des Hinweises auf die Patenterteilung: **10.10.2018**
(21) Anmeldenummer: 14786185.0
(22) Anmeldetag: 17.10.2014
(51) Int. Cl.: C07D 209/86, C07D 209/10, C07F 17/02, B01J 31/24

(54) **MONOARYLIERUNG AROMATISCHER AMINE**
MONO ARYLATION OF AROMATIC AMINES
MONOARYLATION D'AMINES AROMATIQUES

(30) Priorität: 21.10.2013 DE 102013017369; 27.01.2014 EP 14152698; 13.02.2014 EP 14155079
(43) Veröffentlichungstag der Anmeldung: 31.08.2016
(73) Patentinhaber: Umicore AG & Co. KG, 63457 Hanau-Wolfgang (DE)
(72) Erfinder: GOOSSEN, Lukas, 67663 Kaiserslautern (DE); GRUENBERG, Matthias, 67105 Schifferstadt (DE)
(86) Internationale Anmeldenummer: PCT/EP2014/072332
(87) Internationale Veröffentlichungsnummer: WO 2015/059049

(56) Entgegenhaltungen:
- EP-A1- 2 407 502
- EP-A2- 2 421 064
- COLACOT T J ET AL: "Cp2Fe(PR2)2PdCl2 (R=i-Pr, t-Bu) Complexes as Air-Stable Catalysts for Challenging Suzuki Coupling Reactions", ORGANIC LETTERS, AMERICAN CHEMICAL SOCIETY, US, Bd. 6, Nr. 21, 1. Januar 2004 (2004-01-01) , Seiten 3731-3734, XP002620888, ISSN: 1523-7060, DOI: 10.1021/OL048598T [gefunden am 2004-09-17] -& Thomas J Colacot ET AL: "Cp 2 Fe(PR 2 ) 2 PdCl 2 (R = i-Pr, t-Bu) Complexes as Air-Stable Catalysts in Challenging Suzuki Coupling Reactions (Supporting Information)", Organic Letters, 1. Januar 2004 (2004-01-01), Seiten 1-2, XP055121951, Gefunden im Internet: URL:http://pubs.acs.org/doi/suppl/10.1021/ ol048598t/suppl_file/ol048598tsi20040820_0 22606.pdf [gefunden am 2014-06-05]
- OLEG V. GUSEV ET AL: "Palladium Complexes with Metallocene-Bridged Bidentate Diphosphine Ligands: Synthesis, Structure, and Catalytic Activity in Amination and Cross-Coupling Reactions", ORGANOMETALLICS, Bd. 25, Nr. 11, 1. Mai 2006 (2006-05-01), Seiten 2750-2760, XP055121798, ISSN: 0276-7333, DOI: 10.1021/om050869w
- CLAUDIO BIANCHINI ET AL: "Ethylene Carbonylation in Methanol and in Aqueous Media by Palladium(II) Catalysts Modified with 1,1'-Bis(dialkylphosphino)ferrocenes", ORGANOMETALLICS, Bd. 24, Nr. 5, 1. Februar 2005 (2005-02-01), Seiten 1018-1030, XP055121795, ISSN: 0276-7333, DOI: 10.1021/om049109w
- HAMANN B C ET AL: "Sterically hindered chelating alkyl phosphines provide large rate accelerations in palladium-catalyzed amination of aryl iodides, bromides, and chlorides, and the first amination of aryl tosylates [12]", JOURNAL OF THE AMERICAN CHEMICAL SOCIETY, ACS PUBLICATIONS, US, Bd. 120, Nr. 29, 29. Juli 1998 (1998-07-29), Seiten 7369-7370, XP002600782, ISSN: 0002-7863, DOI: 10.1021/JA981318I
- Jens Langer ET AL: "Low-Valent Nickel and Palladium Complexes with 1,1'-Bis(phosphanyl)ferrocenes: Syntheses and Structures of Acrylic Acid and Ethylene Complexes", European Journal of Inorganic Chemistry, vol. 2007, no. 16, 1 June 2007 (2007-06-01), pages 2257-2264, XP55121788, ISSN: 1434-1948, DOI: 10.1002/ejic.200601051

## Beschreibung

Die Hartwig-Buchwald-Reaktion hat mittlerweile Eingang in industrielle Fertigungsprozesse, insbesondere von Zwischenprodukten zur Herstellung organischer Leuchtdioden.

EP-A1-2407502 beschreibt die Herstellung von Dendrimeren für diesen Zweck, wobei sekundäre Amine als Zwischenprodukte durch die Hartwig-Buchwald-Reaktion erhalten werden.

Auch EP-A2-2421064 bedient sich der Hartwig-Buchwald-Reaktion als Syntheseweg für sekundäre Amine, die als Zwischenprodukte genutzt werden.

Hamann et al., J. Am. Chem. Soc. 120, (1998), 7369 zeigt die Verwendung von Bis-(Dialkylphosphinoferrocen)-Liganden in der Hartwig-Buchwald-Reaktion.

Die mäßige Ausbeute ist zwar nachteilig, das größere Problem stellt jedoch die mangelnde Selektivität dar, welche nicht nur zu sekundären, sondern auch tertiären Aminen als Nebenprodukte führt. Diese können nur schwer mittels Sublimation abgetrennt werden. Besonders nachteilig ist dabei außerdem, daß an Zwischenprodukte für die Herstellung von Dendrimeren Zwischenprodukten zur Herstellung organischer Leuchtdioden besonders hohe Reinheitsanforderungen bestehen, da unerwünschte, auch strukturverwandte Verunreinigungen, selbst in geringen Mengen zu einer unerwünschten Verschiebung der Emissionswellenlängen oder Verringerung der Quantenausbeute und damit auch zu unerwünschter Abwärmeentwicklung führen können.

Es ist die Aufgabe der vorliegenden Patentanmeldung, ein Verfahren bereit zu stellen, welches die Herstellung der Dibisphenylaminen unter den Bedingungen der Hartwig-Buchwald-Kupplung in höheren Ausbeuten und Selektivitäten ermöglicht, so dass der Aufwand für die Reinigung der Verbindungen, die meist durch Sublimation erfolgt, verringert werden kann. In Berücksichtigung des oben Erklärten ist es also erforderlich, die Ausbeuteerhöhung vor allem durch Erhöhung der Selektivität zu erreichen.

Die Aufgabe wird gelöst durch ein Verfahren zur selektiven Arylierung eines primären aromatischen Amins der Formel A-NH₂ mit einer aromatischen Verbindung der Formel X-B zu einem sekundären aromatischen Amin A-NH-B, wobei die Reste A und B unabhängig voneinander gleiche oder verschiedene, substituierte oder unsubstituierte aromatische Reste sind und der Rest X eine Abgangsgruppe, insbesondere Halogen, also Fluor, Chlor, Brom, Iod, Astatin, oder ein Trifluormethylsulfonsäurerest ist, wobei in dem sekundären aromatischen Amin die aromatischen Kohlenstoffatome direkt an das Stickstoffatom gebunden sind und mindestens einer der Reste A oder B eine Biphenyleinheit aufweist und die Reaktion in Gegenwart einer Base und eines Palladiumkomplexes durchgeführt wird, wobei das Palladiumatom von einem Bis-(Dialkylphosphinoferrocen)-Liganden komplexiert wird.

### Kurze Beschreibung der Erfindung

Die Erfindung läßt sich in den folgenden Punkten kurz beschreiben:
1. Verfahren zur selektiven Arylierung eines primären aromatischen Amins der Formel A-NH₂ mit einer aromatischen Verbindung der Formel X-B zu einem sekundären aromatischen Amin A-NH-B, wobei die Reste A und B unabhängig voneinander gleiche oder verschiedene, substituierte oder unsubstituierte aromatische Reste sind und der Rest X ein Halogen oder ein Trifluormethylsulfonsäurerest ist, wobei in dem sekundären aromatischen Amin die aromatischen Kohlenstoffatome direkt an das Stickstoffatom gebunden sind und mindestens einer der Reste A oder B eine Biphenyleinheit aufweist und die Reaktion in Gegenwart einer Base und eines Palladiumkomplexes durchgeführt wird, wobei das Palladiumatom von mindestens einem Bis-(Dialkylphosphinoferrocen)-Liganden komplexiert wird.
2. Verfahren nach Punkt 1, wobei die Alkylsubstituenten der Bis-(Dialkylphosphinoferrocen)-Liganden zwei bis fünf Kohlenstoffatome aufweisen.
3. Verfahren nach Punkt 1 oder 2, wobei die Alkylsubstituenten ausgewählt sind aus der Gruppe bestehend aus Isopropyl, Isobutyl, tert.-Butyl und deren Kombinationen.
4. Verfahren nach einem der Punkte 1 bis 3, wobei beide aromatische Substituenten A und B eine Biphenyleinheit aufweisen, welche gleich oder voneinander verschieden sind.
5. Verfahren nach einem der Punkte 1 bis 4, wobei die Biphenyleinheit direkt an das sekundäre Stickstoffatom des Amins gebunden ist.
6. Verfahren nach einem der vorstehenden Punkte, wobei die Alkylsubstituenten ausgewählt sind aus der Gruppe bestehend aus Isopropyl, Isobutyl und deren Kombinationen.
7. Verfahren nach einem der vorstehenden Punkte, wobei die Biphenyleinheit direkt an die Abgangsgruppe, insbesondere ein Halogen, also Chlor, Brom, Iod oder an eine Trifluormethylsulfonsäuregruppe gebunden ist.
8. Verfahren nach einem der vorstehenden Punkte, wobei die Biphenyleinheit eine verbrückte Biphenyleinheit der Formel 2 oder 3 ist. 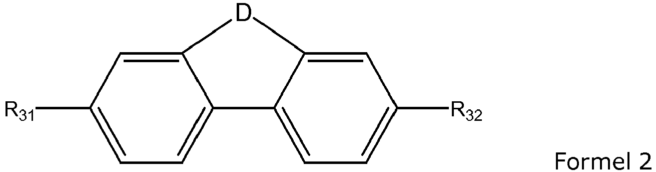 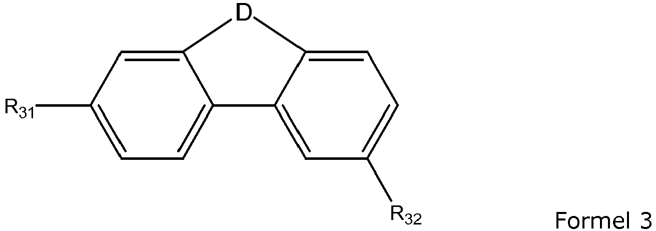 wobei D Sauerstoff, Schwefel, Stickstoff oder Kohlenstoff sein und im Fall von Stickstoff einfach oder im Fall von Kohlenstoff zweifach mit Methyl, Ethyl, Biphenyl, Naphthyl oder Phenyl substituiert sein kann.
9. Verfahren nach einem oder mehreren der vorstehenden Punkte, wobei die Biphenyleinheit A, B oder beide gleich oder verschieden und sind ausgewählt ist aus einer Einheit der Formeln 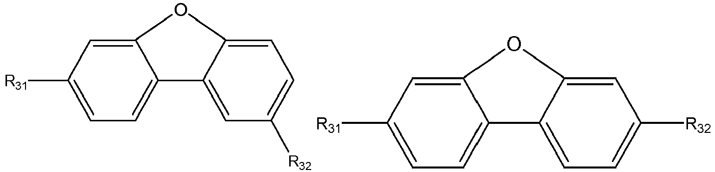 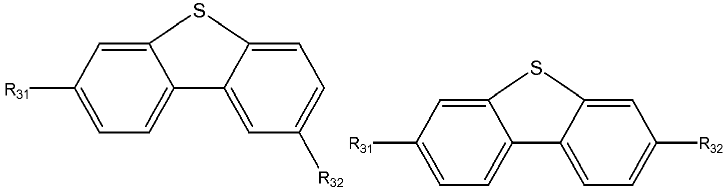 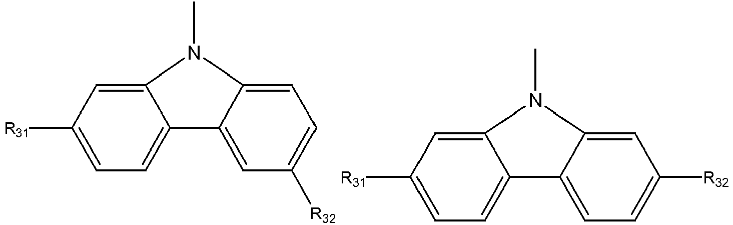 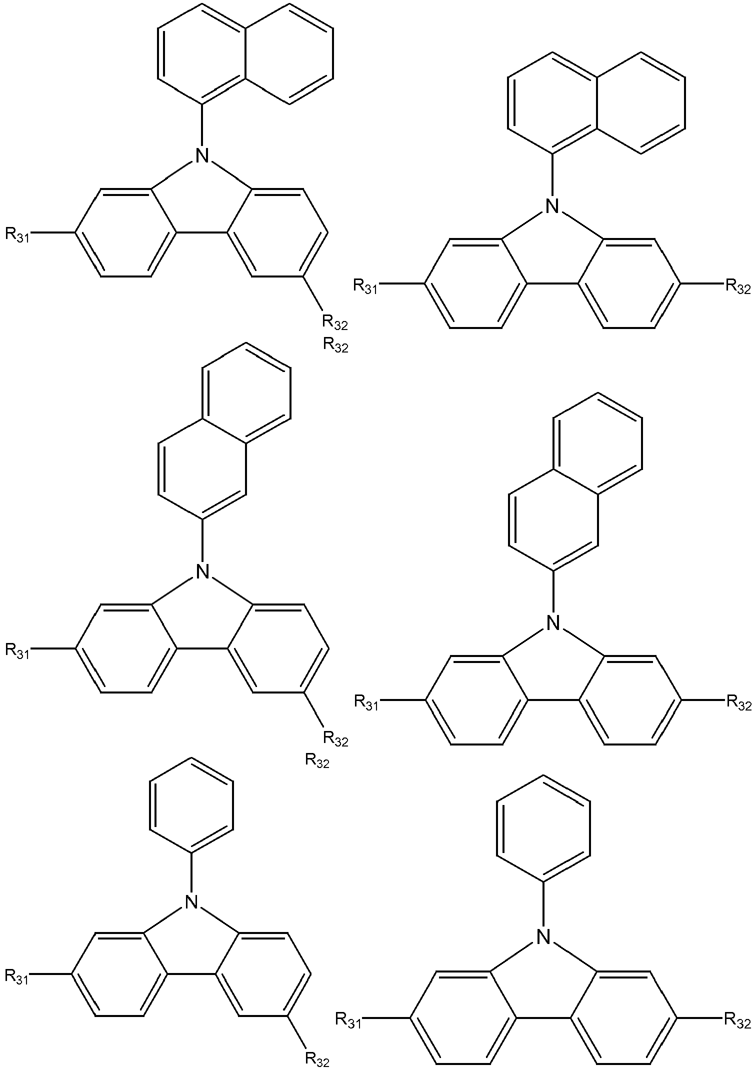 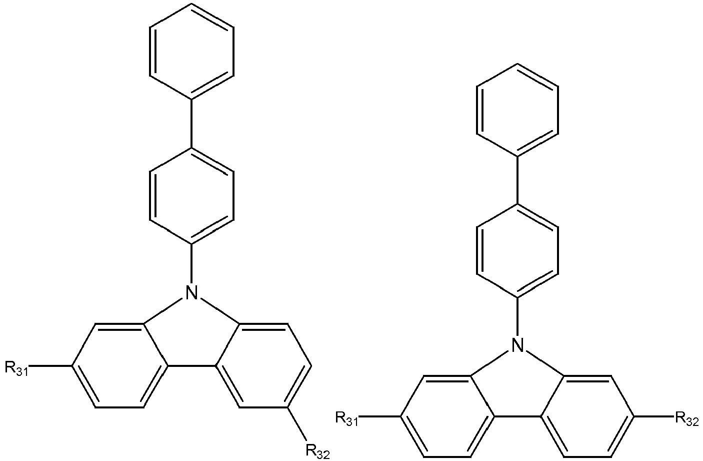 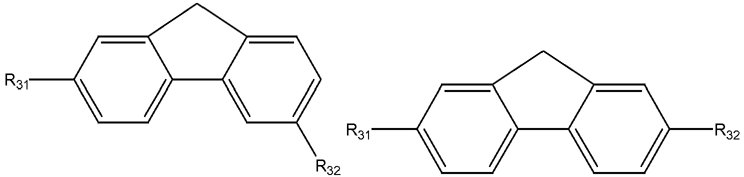 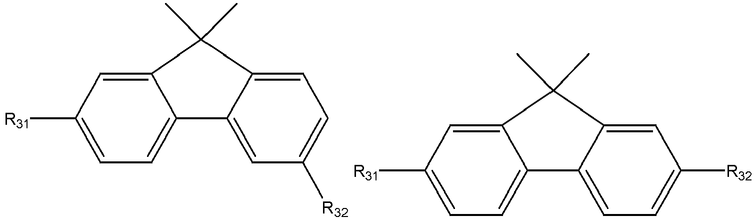 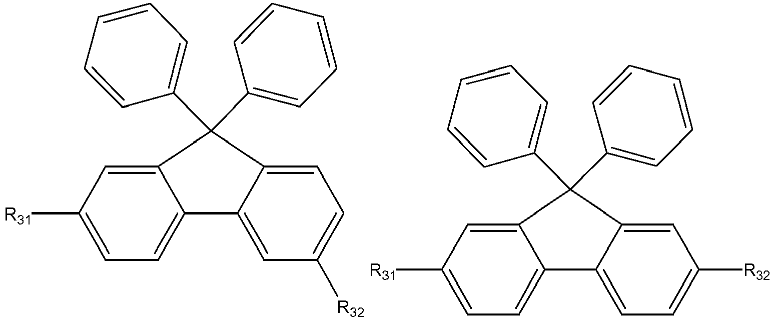 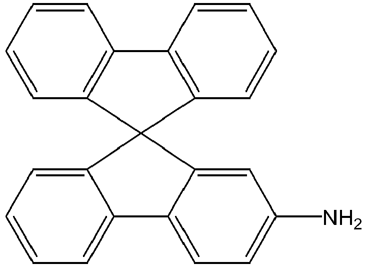 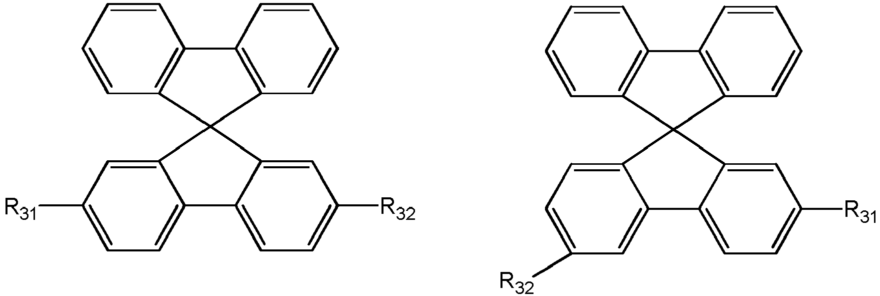 wobei R31 Wasserstoff, Phenyl, Biphenyl oder Pyridyl sein kann,
   R32 eine Abgangsgruppe, Halogen, eine primäre Amingruppe NH₂ oder ein Trifluormethylsulfonsäurerest sein kann, je nachdem ob der Rest als A oder B verwendet wird; oder aber R32 ein Spacer ist, welcher zwischen A bzw. B und X bzw. NH₂ angeodnet ist.
10. Verfahren nach einem oder mehreren der vorstehenden Punkte, wobei der Spacer ausgewählt ist aus der Gruppe bestehend aus 1,4-Phenyl, 1,4-(6-Methyl)phenyl, 1,4-(5-Methyl)phenyl, 4,4'-Biphenyl, 2,6-Naphthyl, 1,4-Naphthyl, 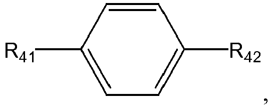 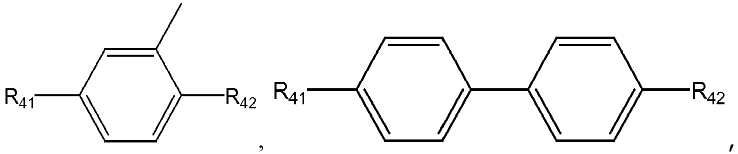 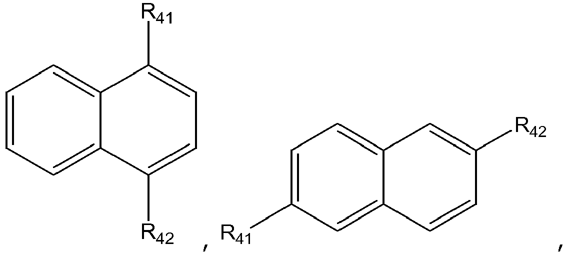 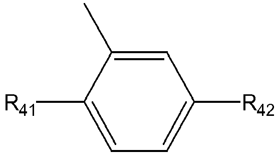 und wobei R41 ist hierbei eine Struktur der Formeln 2 bis 22, R42 kann Halogen wie Fluor, Chlor, Brom, Iod oder Astat, eine primäre Amingruppe NH₂ oder ein Trifluormethylsulfonsäurerest ist.
11. Verfahren nach einem oder mehreren der vorstehenden Punkte, wobei die Base Lithiumhydroxid, Kaliumhydroxid, Natriumhydroxid, tertiäre organische Amine, Alkoholate, Tributylamin, Triethylamin, Alkalimetallalkoholate, Lithiumethanolat, Natriumethanolat, Kaliumethanolat, Lithium-tert.-butanolat, Natrium-tert.-butanolat, Kalium-tert.-butanolat, Lithiumbis(trimethylsilyl)amid, Natriumbis(trimethylsilyl)amid, Kaliumbis(trimethylsilyl)amid oder deren Kombinationen ist.
12. Verfahren nach einem oder mehreren der vorstehenden Punkte, wobei das Verfahren in einem Lösungsmittel ausgewählt aus der Gruppe bestehend aus Alkoholen, Ethanol, Propanol, Isopropanol, Butanol, Isobutanol, Ethylenglykol, Ether, Diethylether, tert.-Butylethylether, tert.-Butylmethylether, Tetrahydrofuran, Dioxan Ethylenglykoldimethylether, Bis(2-methoxyethyl)ether, aromatische Lösungsmittel, Benzol, Toluol, Xylol, o-Xylol, p-Xylol, m-Xylol und deren Kombinationen durchgeführt wird.
13. Verfahren nach einem oder mehreren der vorstehenden Punkte, wobei das Verfahren bei einer Temperatur von 5°C bis 150°C, 20°C bis 140°C, 30°C bis 130°C, 60°C bis 120°C oder 70°C bis 111°C durchgeführt wird.
14. Verfahren nach einem oder mehreren der vorstehenden Punkte, wobei die Reaktionszeit des Verfahrens von einer Stunde bis 36 Stunden, 4 Stunden bis 24 Stunden, 6 Stunden bis 16 Stunden oder 8 Stunden bis 12 Stunden liegt.
15. Verfahren nach einem oder mehreren der vorstehenden Punkte, wobei die Biphenyleinheit 2-Fluoren, 3-Fluoren, 2-(9,9-Diphenylfluoren), 2-(9,9-Dimethylfluoren), 3-(9,9-Diphenylfluoren), 3-(9,9-Dimethylfluoren), 3-(4-phenyl)-9-phenyl-9H-carbazol, 3-(4-phenyl)-9-methyl-9H-carbazol, 3-(4-phenyl)-9-Biphenyl-9H-carbazol, 2-(4-phenyl)-9-phenyl-9H-carbazol, 2-(4-phenyl)-9-methyl-9H-carbazol, 2-(4-phenyl)-9-Biphenyl-9H-carbazol, 3-(4-Biphenyl)-9-phenyl-9H-carbazol, 3-(4-Biphenyl)-9-methyl-9H-carbazol, 3-(4-Biphenyl)-9-Biphenyl-9H-carbazol, 2-(4-Biphenyl)-9-phenyl-9H-carbazol, 2-(4-Biphenyl)-9-methyl-9H-carbazol, 2-(4-Biphenyl)-9-Biphenyl-9H-carbazol, -- 3-(9-phenyl-9H-carbazol), 3-(9-methyl-9H-carbazol), 3-(9-Biphenyl-9H-carbazol), 2-(9-phenyl-9H-carbazol), 2-(9-methyl-9H-carbazol), 2-(9-Biphenyl-9H-carbazol) oder Triphenylene ist.
16. Verfahren nach einem oder mehreren der vorstehenden Punkte, wobei der Palladiumkomplex in Mengen von 0,01 mol% bis 1,5 mol%, bezogen auf die molare Gesamtmenge an beiden Edukten, dem primären aromatischen Amin A-NH₂ und dem Halogenaromaten X-B, eingesetzt wird.
17. Verfahren nach einem oder mehreren der vorstehenden Punkte, wobei der Palladiumkomplex als Feststoff, Lösung oder Pulvermischung mit einem festen Bis-(Dialkylphosphinoferrocen) verwendet wird.
18. Verfahren nach einem der vorstehenden Punkte enthaltend die Schritte:
   - Bereitstellen des primären aromatischen Amins A-NH₂, des Halogenaromaten X-B, eines geeigneten Lösemittels und gegebenenfalls eines Bis-(Dialkylphosphinoferrocens) in einem Reaktionsgefäß;
   - Zugeben eines Palladiumkomplexes, bei welchem das Palladiumatom von mindestens einem Bis-(Dialkylphosphinoferrocen)-Liganden komplexiert wird, in Form eines Feststoffs oder einer Lösung;
   - Erwärmen des so erhaltenen Reaktionsgemisches in dem Reaktionsgefäß;
   - Abtrennen des Reaktionsproduktes, eines sekundären aromatischen Amin A-NH-B; und
   - Gegebenenfalls Reinigung des sekundären aromatischen Amin A-NH-B.
19. Verfahren nach einem oder mehreren der vorstehenden Punkte, wobei die Verbindungen 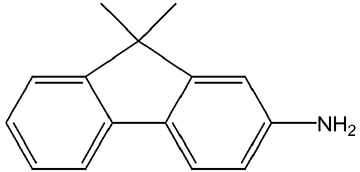 und 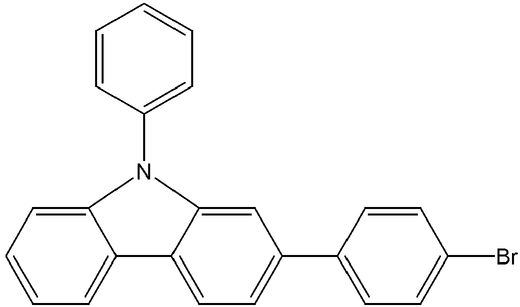 vorhanden sind.
20. Verfahren nach einem oder mehreren der vorstehenden Punkte, wobei in dem Palladiumkomplex das Palladiumatom zusätzlich von 2,4,6,8-Tetramethylcyclotetrasiloxan, Bis(dibenzylidenaceton) oder Maleimid komplexiert wird.
21. Verfahren nach einem oder mehreren der vorstehenden Punkte, wobei das Palladiumatom in dem Palladiumkomplex von 1,1'-Bis(diisopropylphosphino)ferrocen komplexiert wird.
22. Die chemische Verbindung 1,1'-Bis(diisopropylphosphino)ferrocenpalladium(0)-maleimid [Pd(dippf)(maleimid)]; 1,1'-Bis(diisopropylphosphino)ferrocenpalladium(0)-1,3-Divinyl-1,1,3,3,-tetramethyldisiloxan[Pd(dippf)(VTS)]; 1,1'-Bis(diisopropylphosphino)ferrocenpalladium(0)-bis(dibenzylidenaceton) [Pd(dippf)(dba)]; 1,1'-Bis(diisopropylphosphino)ferrocenpalladium(0)- naphthochinon [Pd(dippf)(Naphthochinon)]; 1,1'-Bis(diisopropylphosphino)ferrocenpalladium(0)-maleinsäureanhydrid [Pd(dippf)(Maleinsäureanhydrid)] oder 1,1'-Bis(diisopropylphosphino)ferrocenpalladium(0)-maleinsäurediethylester [Pd(dippf)(Maleinsäurediethylester)].
23. Verwendung einer Verbindung 1,1'-Bis(diisopropylphosphino)ferrocenpalladium(0)-maleimid [Pd(dippf)(maleimid)]; 1,1' Bis(diisopropylphosphino)ferrocenpalladium(0)-1,3-Divinyl-1,1,3,3,-tetramethyldisiloxan [Pd(dippf)(VTS)]; 1,1'Bis(diisopropylphosphino)ferrocenpalladium(0)-bis(dibenzylidenaceton) [Pd(dippf)(dba)]; 1,1'Bis(diisopropylphosphino)ferrocenpalladium(0)-naphthochinon[Pd(dippf) (Naphthochinon)]; 1,1'Bis(diisopropylphosphino)ferrocenpalladium(0)-maleinsäureanhydrid [Pd(dippf)(Maleinsäureanhydrid)]; 1,1'Bis(diisopropylphosphino)ferrocenpalladium(0)-maleinsäurediethylester [Pd(dippf)(Maleinsäurediethylester)] oder 1,1'-Bis(diisopropylphosphino)ferrocenpalladium(0)-Norbornen [Pd(dippf) (Norbornen)] als Katalysator für die Hartwig-Buchwald Kupplung.

### Detaillierte Beschreibung der Erfindung

In dem Palladiumkomplex wird das Palladiumatom von mindestens einem Bis-(Dialkylphosphinoferrocen)-Liganden komplexiert, welcher die allgemeine Formel 1 aufweist: 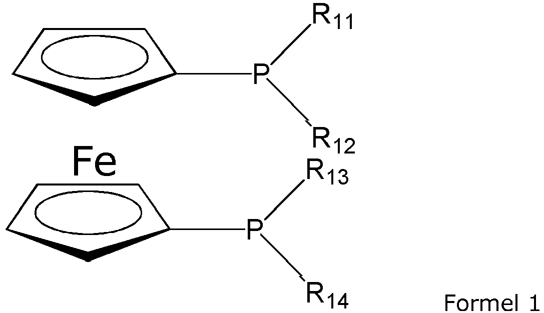 R11 bis R14 können gleich oder verschieden sein und sind insbesondere Alkylreste mit einem bis fünf Kohlenstoffatomen. R11 bis R14 können also unabhängig voneinander ausgewählt sein aus Methyl, Ethyl, n-Propyl, Isopropyl, n-Butyl, Isobutyl, sec.-butyl, tert.-Butyl, n-Pentyl (Amyl), 2-Pentyl (sec-Pentyl), 3-Pentyl, 2-Methylbutyl, 3-Methylbutyl (iso-Pentyl oder iso-Amyl), 3-Methylbut-2-yl, 2-Methylbut-2-yl,2,2-Dimethylpropyl (Neopentyl).

Vorteilhaft sind R11 bis R14 gleich und ausgewählt aus der Gruppe bestehend aus Methyl, Ethyl, n-Propyl, Isopropyl, n-Butyl, Isobutyl, sec.-butyl, tert.-Butyl, n-Pentyl (Amyl), 2-Pentyl (sec-Pentyl), 3-Pentyl, 2-Methylbutyl, 3-Methylbutyl (iso-Pentyl oder iso-Amyl), 3-Methylbut-2-yl, 2-Methylbut-2-yl,2,2-Dimethylpropyl (Neopentyl).

Besonders geeignet sind Isopropyl, Isobutyl, tert.-Butyl, insbesondere Isopropyl und tert.-Butyl. Gute Ergebnisse werden erzielt, wenn R11 bis R14 gleich und Isopropyl oder tert.-Butyl sind. Gute Ergebnisse werden insbesondere erzielt, wenn R11 bis R14 gleich und Isopropyl, Propyl oder Isobutyl sind. Gute Ergebnisse werden insbesondere erzielt, wenn R11 bis R14 gleich und Isopropyl sind.

Es wurde überraschend gefunden, dass Bis(Dialkylphosphinoferrocen)-Liganden der Formel 1 bei der Herstellung von sekundären Aminen durch Hartwig-Buchwald-Kupplung die selektive Herstellung der sekundären Amine mit hohen Ausbeuten gestatten, wenn zumindest einer der Reste A oder B der zu kuppelnden Verbindungen als Strukturelement mindestens eine Biphenyleinheit aufweist. Hierbei kann die verwendete Palladiummenge von den normalerweise verwendeten 3 mol% auf etwa 1,5 mol% oder weniger gesenkt werden, wobei die normalerweise als Nebenreaktion auftretende Doppelarylierung zum tertiären Amin unterdrückt wird und die Reaktion zum gewünschten Produkt mit Umsätzen von meist 90% oder mehr abläuft.

Vorzugsweise weisen beide Reste A und B eine Biphenyleinheit auf. Die Biphenyleinheit kann unsubstituiert oder substituiert sein, auch mit einem oder mehreren Phenylresten, so dass beispielsweise Terphenyl-, Quaterphenyl- oder Triphenyleneinheiten entstehen, die substituiert oder unsubstituiert sein können. Die Biphenyleinheiten können auch verbrückt sein, wie beispielsweise im Fall von Fluoren und dessen Derivaten.

Als Biphenyl können insbesondere die Verbindungen der Formeln 2 oder 3 als Reste A oder B eingesetzt werden:

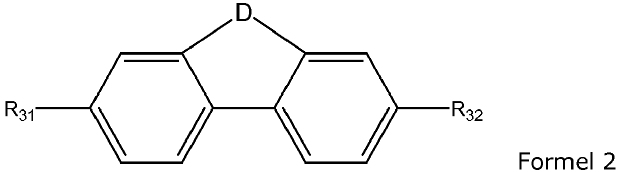

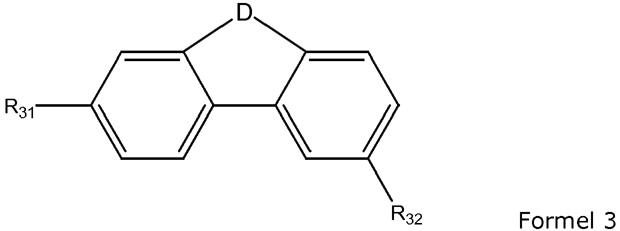

D kann Sauerstoff, Schwefel, Stickstoff oder Kohlenstoff sein und einfach (im Fall von Stickstoff) oder zweifach (im Fall von Kohlenstoff) mit Methyl, Ethyl, Biphenyl, 1-Naphthyl, 2-Naphthyl oder Phenyl substituiert sein. Es können also insbesondere die folgenden Reste vorliegen:

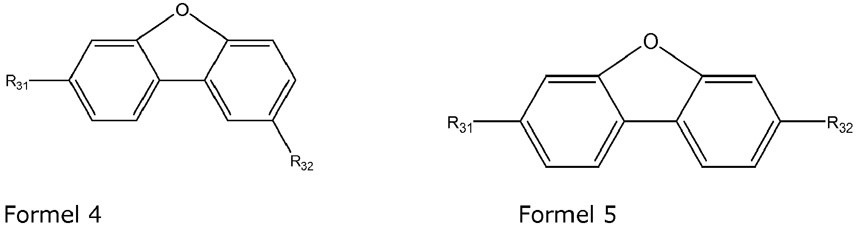

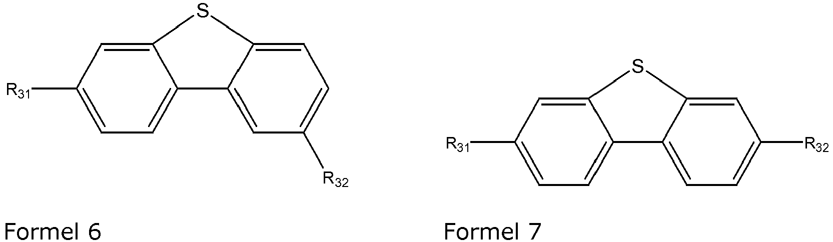

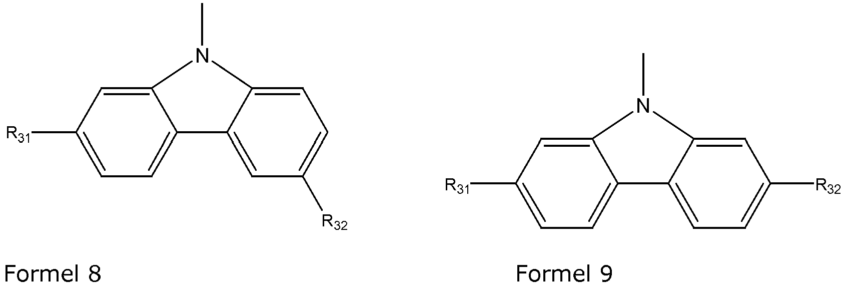

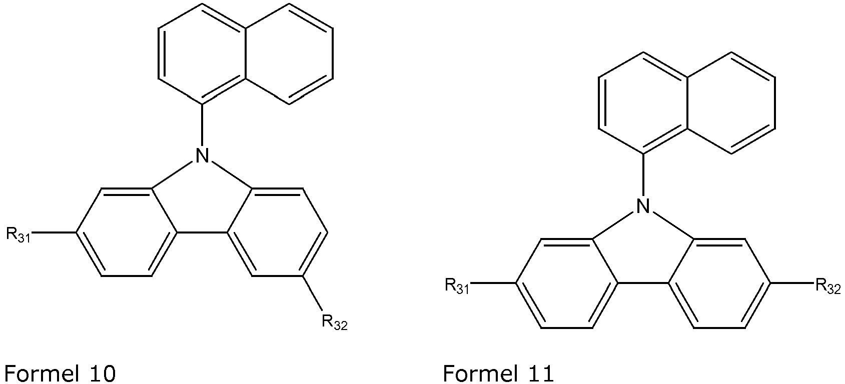

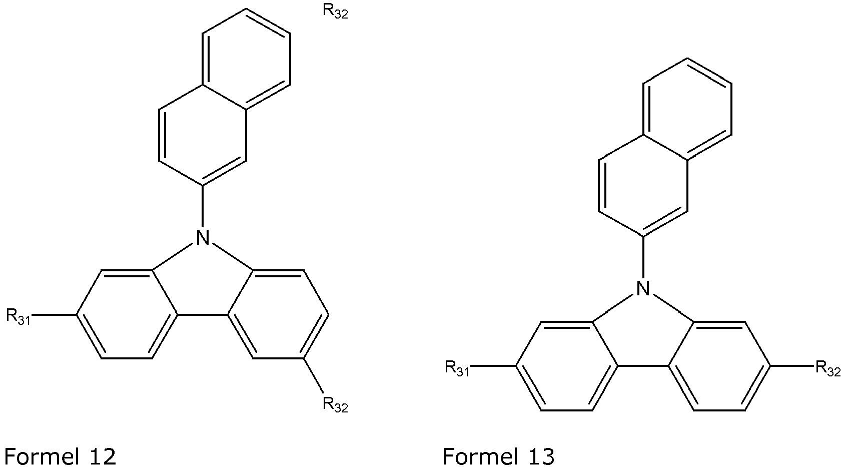

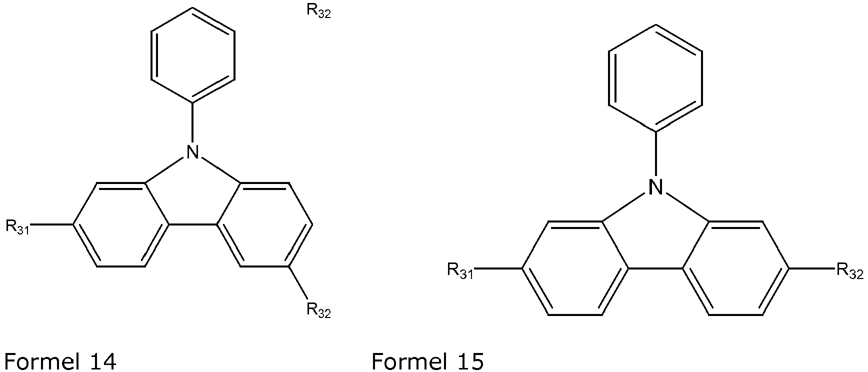

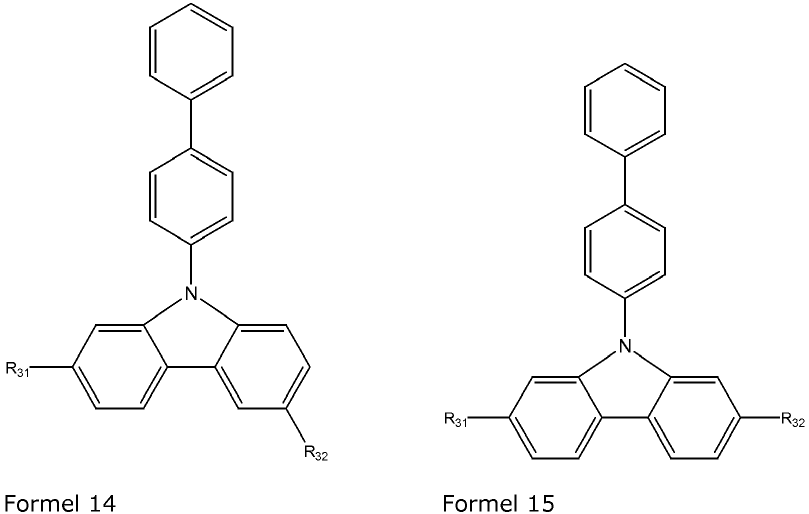

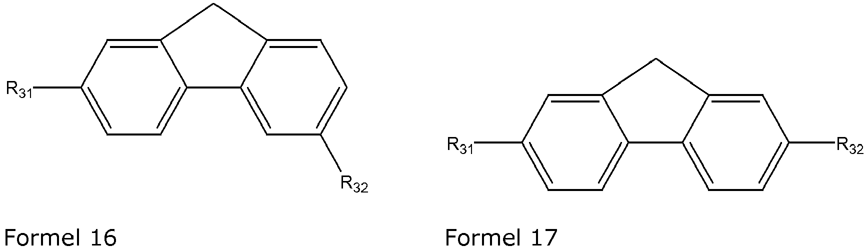

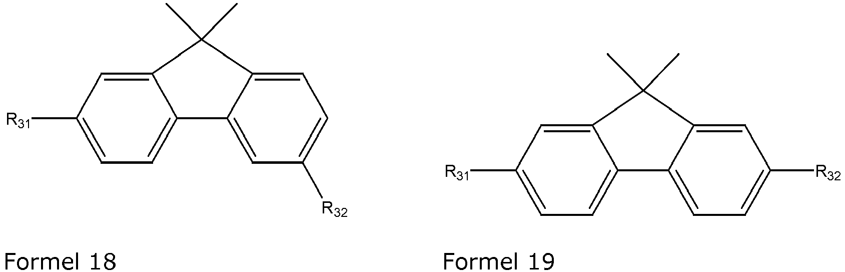

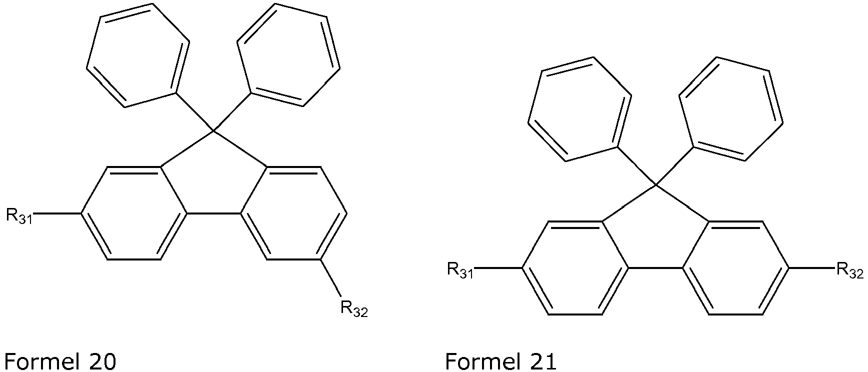

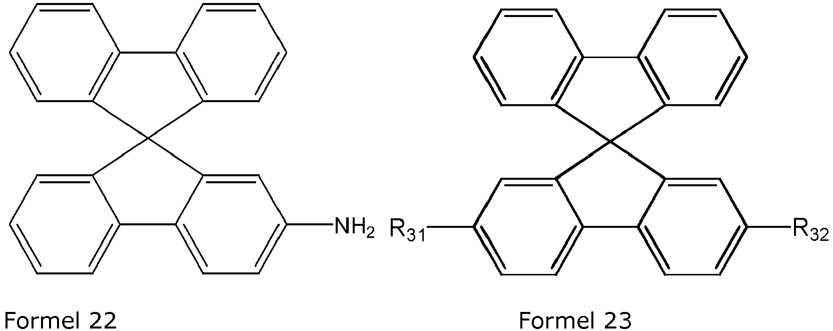

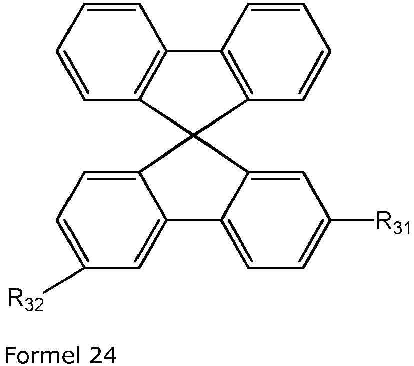

R31 kann Wasserstoff, Phenyl, Biphenyl oder Pyridyl sein.

R32 kann entweder wie oben beschrieben Halogen, eine primäre Amingruppe NH₂ oder ein Trifluormethylsulfonsäurerest sein, je nachdem ob der Rest der Formel 2 oder 3 als A oder B verwendet wird.

R32 kann aber auch ein Spacer sein, welcher zwischen A bzw. B und X bzw. NH₂ angeodnet ist. Geeignete Spacer sind beispielsweise 1,4-Phenyl, 1,4-(6-Methyl)phenyl, 1,4-(5-Methyl)phenyl, 4,4'-Biphenyl, 2,6-Naphthyl oder 1,4-Naphthyl. Dies sind insbesondere

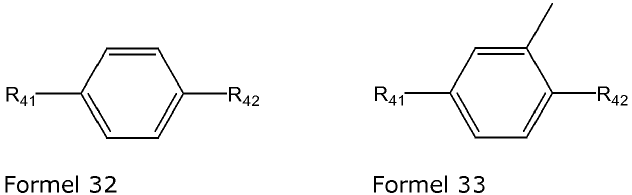

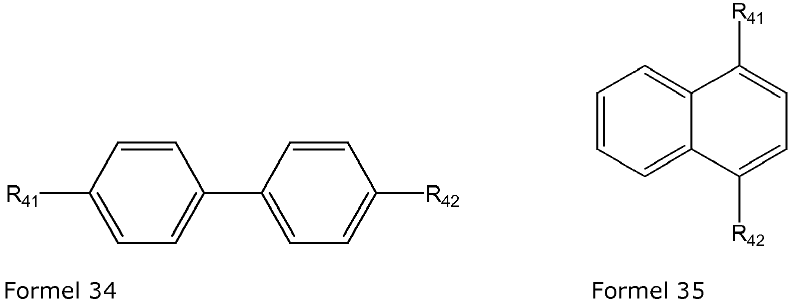

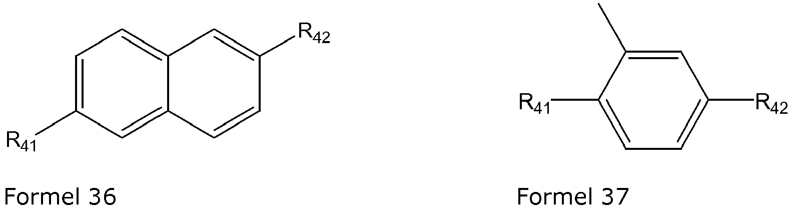

R41 ist hierbei eine Struktur der Formeln 2 bis 22, R42 kann Halogen wie Fluor, Chlor, Brom, Iod oder Astat, eine primäre Amingruppe NH₂ oder ein Trifluormethylsulfonsäurerest sein.

In einer spezifischen Ausgestaltung der Erfindung werden die folgenden Verbindungen verwendet, die Verbindung der Formel 131 als Amin der Formel A-NH₂ und die Verbindung der Formel 132 als Halogenaromat der Formel B-X

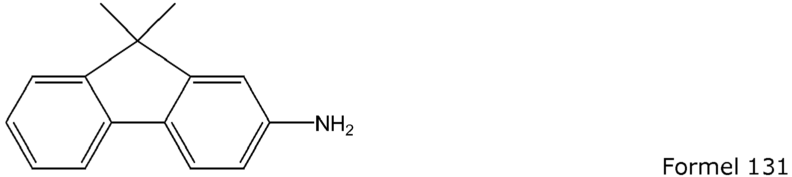

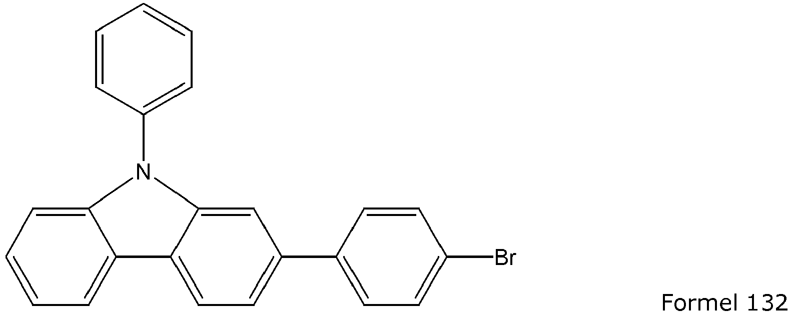

Die Ausgangsverbindungen, das primäre aromatische Amin A-NH₂ und der Halogenaromat X-B, werden äquimolar eingesetzt. Gegebenenfalls kann der Halogenaromat oder das Amin auch in einem Überschuß bis zum 1,1-fachen oder 1,2-fachen des äquimolaren Verhältnisses eingesetzt werden.

Der Palladiumkomplex kann auf im Prinzip bekannte Weise erhalten werden. Hierzu wird zunächst eine Palladiumverbindung in einem Lösungsmittel vorgelegt und anschließend der gewünschte Bis-(Dialkylphosphinoferrocen)-Ligand zugegeben, wobei mit 1,1'-Bis(diisopropylphosphino)ferrocen, 1,1'-Bis(diisobutylphosphino)ferrocen, 1,1'-Bis(di-tert.-butylphosphino)ferrocen gute Ergebnisse erzielbar sind. Anschließend kann zwischen 30 und 1000 Minuten, insbesondere 40 bis 400 Minuten oder 50 bis 120 Minuten gerührt werden. Die Reaktionstemperatur kann von etwa 10°C bis 100°C, insbesondere 15°C bis 50°C oder 20°C bis 30°C betragen.

Gute Ergebnisse lassen sich bereits durch Rühren bei Raumtemperatur für etwa eine Stunde erzielen.

Die Obergrenze der Temperatur hängt im Wesentlichen von der Siedetemperatur des Lösungsmittels ab, so dass hochsiedende Lösungsmittel für höhere Reaktionstemperaturen erforderlich sind und die oben angegebenen Temperaturobergrenzen nicht als starr, sondern in Abhängigkeit zur Siedetemperatur des Lösungsmittels zu sehen sein können.

Geeignete Lösungsmittel sind im Allgemeinen aprotische Lösungsmittel, wie Ether oder aromatische Lösungsmittel. Geeignet sind daher beispielsweise Diethylether, tert.-Butylethylether, tert.-Butylmethylether, Tetrahydrofuran oder Dioxan, aber auch Benzol, Toluol oder Xylol, aber auch Acetonitril kann eingesetzt werden.

Gute Ergebnisse sind durch Verwendung möglichst wasser- und sauerstofffreier Lösungsmittel erreichbar, die durch übliche Trocknungsverfahren der Lösemittel erhalten werden können.

Als Edukt für den Palladiumkomplex geeignete Palladiumverbindungen können Pd (0) und Pd(II)-Komplexe sein, wie z.B. Allylchloro[1,3-bis(2,4,6-trimethylphenyl)imidazol-2-ylidene]palladium(II), (Ethylenediamine) palladium(II)chlorid, Palladium(II) acetat, Palladium(II) chlorid, Palladiumpivalat, Palladium(II)acetylacetonat, Bis(benzonitrile)palladium(II) chloride, Bis(acetonitrile)dichlorpalladium(II), Diammindichlorpalladium(II), Dichlor(1,5-cyclooctadiene)palladium(II), Palladium(II)nitrat, Palladium(II)oxid, Palladium(II)oxidhydrat, H₂[PdCl₄], Diammindinitritopalladium(II), Palladium(II)sulfate, Tetraamminpalladium(II)sulfat [Pd(NH₃)₄]SO₄Tetraamminpalladium(II) hydrogencarbonat, Tetraamminpalladium(II)chlorid [Pd(NH₃)₄]Cl₂, Kaliumtetrachlorpalladat(II) K₂[PdCl₄], Natriumtetrachlorpalladat(II) Na₂[PdCl₄], Ammoniumtetrachlorpalladate(II) (NH₄)₂[PdCl₄], Tetraamminpalladium(II)nitrat, 1,3-Divinyl-1,1,3,3-tetramethyldisiloxanpalladium(0) (auch als Pd-VTS, Pd-VS oder Palladium-VTS), Bis(dibenzylidenaceton)palladium(0) (Pd(dba)₂), (Pd₂(dba)₃), (Pd₂(dba)₃)·LM, wobei LM ein Lösungsmittel, insbesondere CHCl₃ oder CH₂Cl₂ ist.
Auf diese Weise wird eine Katalysatorlösung erhalten.

Nach der Herstellung des Palladiumkomplexes kann weiter verfahren werden, indem im Sinne einer Ein-Topf-Reaktion die weiteren Reaktanden, also das primäre aromatische Amin A-NH₂, der Halogenaromat X-B und die für die Hartwig-Buchwald-Kupplung erforderliche Base zu der Katalysatorlösung zu gegeben werden. Als Base sind Alkali- und Erdalkalimetallhydroxide geeignet, wie beispielsweise Lithiumhydroxid, Kaliumhydroxid oder Natriumhydroxid. Ebenso geeignet sind tertiäre organische Amine oder Alkoholate, wie Tributylamin, Triethylamin, Alkalimetallalkoholate wie Lithiumethanolat, Natriumethanolat oder Kaliumethanolat, Lithium-tert.-butanolat, Natrium-tert.-butanolat oder Kalium-tert.-butanolat, Lithiumbis(trimethylsilyl)amid, Natriumbis(trimethylsilyl)amid, Kaliumbis(trimethylsilyl)amid, einzeln oder in Kombination miteinander, als Base geeignet.

Die Base kann in Bezug auf die gesamte molare Menge beider Edukte, des primären aromatischen Amins A-NH2 und des Halogenaromaten X-B, im Allgemeinen in Mengen von 40% bis 80%, insbesondere 50% bis 70% oder 55% bis 65% eingesetzt werden. Das bedeutet, wenn z.B. je 20 mmol des primären aromatischen Amins A-NH2 und des Halogenaromaten X-B, zusammen also 40 mmol, verwendet werden, so kann die Base in Mengen von 16 mmol bis 32 mmol, insbesondere 20 mmol bis 28 mmol oder von 22 mmol bis 26 mmol eingesetzt werden.

Alternativ zu dieser Vorgehensweise können auch das primäre aromatische Amin A-NH₂, der Halogenaromat X-B und die Base mit einem Lösemittel versehen vorgelegt werden und die Katalysatorlösung in entsprechender Menge zudosiert werden.

Als Lösungsmittel für die Kupplungsreaktion zwischen dem aromatische Amin A-NH₂ und dem Halogenaromaten X-B geeignet sind die üblichen Lösungsmittel für Hartwig-Buchwald-Kupplungen, also Alkohole wie Ethanol, Propanol, Isopropanol, Butanol, Isobutanol, aber auch z.B. Ethylenglykol, Ether wie Diethylether, tert.-Butylethylether, tert.-Butylmethylether, Tetrahydrofuran, Dioxan Ethylenglykoldimethylether, Bis(2-methoxyethyl)ether, aromatische Lösungsmittel wie Benzol, Toluol oder Xylole, wie o-Xylol, p-Xylol, m-Xylol sowie deren Gemische, oder Kombinationen dieser Lösemittel. Bei den Lösemitteln ist darauf zu achten, dass diese unter den Bedingungen der Hartwig-Buchwald-Kupplung inert sind, da Amino- oder Halogengruppen die gewünschte Reaktion stören können.

Die Katalysatormenge, die für die Umsetzung ohne die als Nebenreaktion auftretende Doppelarylierung zum tertiären Amin nötig ist, liegt zwischen 0,01% bis 1,5 mol%, oder von 0,1 bis 1 mol%, oder von 0,3 mol% bis 0,8 mol%. Die Angabe in mol% bezieht sich auf die Gesamtmenge in mol beider Edukte, des primären aromatischen Amins A-NH₂ und des Halogenaromaten X-B.

Die Umsätze der Edukte liegen mindestens bei 90%, insbesondere bei mindestens 95% oder bei mindestens 97%. Die oft als Nebenreaktion auftretende Doppelarylierung zum tertiären Amin wird hierbei fast vollständig unterdrückt.

Die Reaktionstemperaturen liegen im Allgemeinen bei 5°C bis 150°C, oder 20°C bis 140°C, oder 30°C bis 130°C, insbesondere 60°C bis 120°C oder 70°C bis 111°C. Die Reaktionszeiten liegen im Allgemeinen bei einer Stunde bis 36 Stunden, oder 4 Stunden bis 24 Stunden oder 6 Stunden bis 16 Stunden oder 8 Stunden bis 12 Stunden.

In einer vorteilhaften Ausgestaltung wird neben dem Palladiumkomplex, wobei das Palladiumatom mit einem Bis-(Dialkylphosphinoferrocen)-Liganden der Formel 1 komplexiert ist als Katalysator zusätzlich noch der entsprechende Ligand, also der im Palladiumkomplex verwendete Bis-(Dialkylphosphinoferrocen)-Ligand, zugegeben. Hier hat sich eine Zugabe im Verhältnis von Palladiumkomplex zu Bis-(Dialkylphosphinoferrocen)-Ligand im Bereich von 1 zu 10 bis 10 zu 1, oder von 1:5 bis 5:1, insbesondere von 2,5:1 bis 1:2,5, wie zum Beispiel 2 zu 1, als sinnvoll herausgestellt. Das Verhältnis bezieht sich auf die molaren Mengen von Palladiumkomplex und Bis-(Dialkylphosphinoferrocen)-Ligand. Dies bedeutet zum Beispiel, dass bei Verwendung von 0,2 mol% Palladiumkomplex 0,1 mol% Bis-(Dialkylphosphinoferrocen)-Ligand zugegeben werden.

Bei der oben beschriebenen Vorgehensweise kann die zusätzliche Menge Bis-(Dialkylphosphinoferrocen)-Ligand beispielsweise zu der Katalysatorlösung zugegeben werden, sowohl vor, während oder nach deren Herstellung aus den Ausgansprodukten, und diese dann bis zur Durchführung der Reaktion gelagert werden.

Alternativ können auch der Bis-(Dialkylphosphinoferrocen)-Ligand, das primäre aromatische Amin A-NH₂, der Halogenaromat X-B und die Base mit einem Lösemittel versehen gemeinsam vorgelegt werden und die Katalysatorlösung in entsprechender Menge zudosiert werden.

Alternativ kann aus der Katalysatorlösung aber auch ein fester Katalysator hergestellt werden. Hierzu wird der Katalysatorlösung ein Additiv zugegeben, welches einen schwer löslichen und gut kristallisierenden Palladiumkomplex bildet. Hierbei wird eine stöchiometrische Verbindung präzipitiert. Diese ist unter den Bedingungen der Präzipitation, also bei ausreichend niedrigem Lösemittelvolumen, ausreichend schwer löslich um auszufallen, jedoch gut genug löslich um wieder zur Herstellung einer Katalysatorlösung verwendet zu werden.

Bei der Herstellung des Palladiumkomplexes wird das Palladiumatom neben dem Bis-(Dialkylphosphinoferrocen)-Liganden auch von Liganden aus der zur Herstellung des Palladiumkomplexes eingesetzten Palladiumverbindung komplexiert, also beispielsweise von 1,3-Divinyl-1,1,3,3-tetramethyldisiloxan (auch als VTS oder VS bekannt), Bis(dibenzylidenaceton) (dba) oder Anderen.

Diese Palladiumkomplexe sind jedoch gut löslich. Aufgabe des Additivs ist es, das Palladiumatom zu komplexieren und hierdurch einen schwerer löslichen Palladiumkomplex zu bilden, der leicht abgetrennt und weiter verarbeitet werden kann. Geeignet als Additiv sind beispielsweise Naphthochinon, Maleinsäureanhydrid, Maleimid, Maleinsäurediethylester oder Norbornen, insbesondere Naphthochinon oder Maleimid. Es bilden sich dann schwer lösliche, gut kristallisierende Palladiumkomplexe, welche abfiltriert, gewaschen und getrocknet werden können. Hierbei wird eine stöchiometrische Verbindung präzipitiert. Diese ist unter den Bedingungen der Präzipitation, also bei ausreichend niedrigem Lösemittelvolumen, ausreichend schwer löslich um auszufallen, jedoch gut genug löslich um wieder zur Herstellung einer Katalysatorlösung verwendet zu werden.

Daher betrifft die vorliegende Patentanmeldung auch die Stoffe 1,1'-Bis(diisopropylphosphino)ferrocenpalladium(0)-maleimid [Pd(dippf)(maleimid)], 1,1'-Bis(diisopropylphosphino)ferrocenpalladium(0)-1,3-Divinyl-1,1,3,3,-tetramethyldisiloxan [Pd(dippf)(VTS)], 1,1'-Bis(diisopropylphosphino)ferrocenpalladium(0)-bis(dibenzylidenaceton) [Pd(dippf)(dba)], 1,1'-Bis(diisopropylphosphino)ferrocenpalladium(0)-naphthochinon [Pd(dippf)(Naphthochinon)], 1,1'-Bis(diisopropylphosphino)ferrocenpalladium(0)-maleinsäureanhydrid [Pd(dippf)(Maleinsäureanhydrid)], 1,1'-Bis(diisopropylphosphino)ferrocenpalladium(0)-maleinsäurediethylester [Pd(dippf)(Maleinsäurediethylester)], 1,1'-Bis(diisopropylphosphino)ferrocenpalladium(0)-Norbornen [Pd(dippf)(Norbornen)].

Das Palladiumatom (Palladium in der Oxidationsstufe 0) ist oft dreifach koordiniert und die Komplexe in der Regel trigonal-planar, so daß Verbindungen wie 1,3-Divinyl-1,1,3,3-tetramethyldisiloxan (VTS oder VS) oder Bis(dibenzylidenaceton), welche mehr als eine Doppelbindung enthalten, das Palladiumatom nur mit einer ihrer Doppelbindungen komplexieren. Die zweite Doppelbindung komplexiert entweder nicht oder ein weiteres Palladiumatom.

Die Struktur einiger dieser Verbindungen kann wie folgt dargestellt werden:

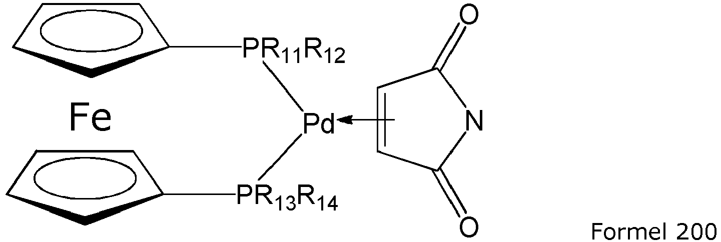

Die Reste R11 bis R14 können in Formel 200 gleich oder verschieden sein wie oben beschrieben und sind insbesondere Alkylreste mit einem bis fünf Kohlenstoffatomen. Sind R11 bis R14 Isopropylreste, so zeigt Formel 200 1,1'-Bis(diisopropylphosphino)ferrocenpalladium(0)-maleimid [Pd(dippf)(maleimid)].

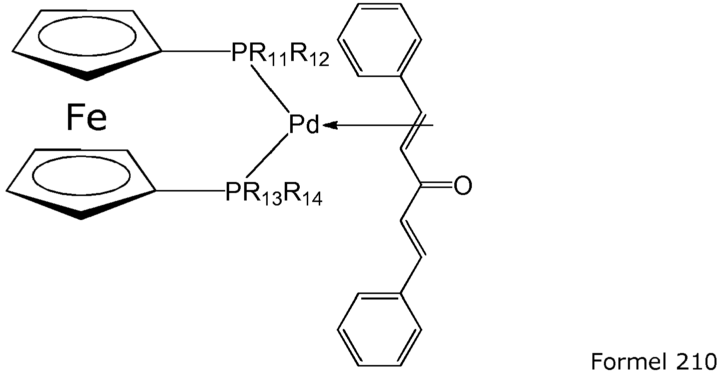

Die Reste R11 bis R14 können in Formel 210 gleich oder verschieden sein wie oben beschrieben und sind insbesondere Alkylreste mit einem bis fünf Kohlenstoffatomen. Sind R11 bis R14 Isopropylreste, so zeigt Formel 210 1,1'-Bis(diisopropylphosphino)ferrocenpalladium(0)-bis(dibenzylidenaceton) [Pd(dippf)(dba)].

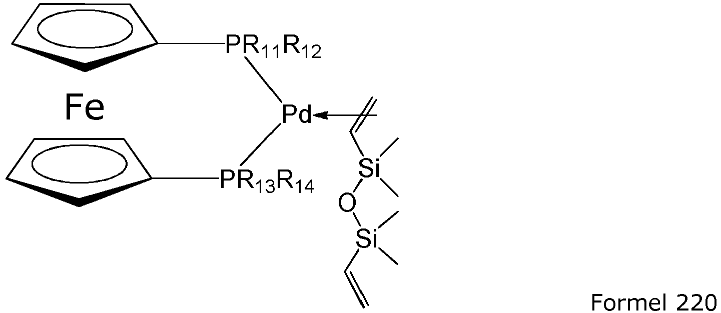

Die Reste R11 bis R14 können in Formel 220 gleich oder verschieden sein wie oben beschrieben und sind insbesondere Alkylreste mit einem bis fünf Kohlenstoffatomen. Sind R11 bis R14 Isopropylreste, so zeigt Formel 220 1,1'-Bis(diisopropylphosphino)ferrocenpalladium(0)-1,3-Divinyl-1,1,3,3,-tetramethyldisiloxan [Pd(dippf)(VTS)].

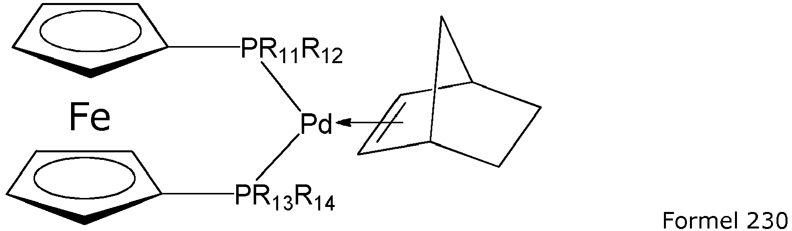

Die Reste R11 bis R14 können in Formel 230 gleich oder verschieden sein wie oben beschrieben und sind insbesondere Alkylreste mit einem bis fünf Kohlenstoffatomen. Sind R11 bis R14 Isopropylreste, so zeigt Formel 230 1,1'-Bis(diisopropylphosphino)ferrocenpalladium(0)-Norbornen [Pd(dippf)(Norbornen)].

Diese festen Palladiumkomplexe können ebenfalls wie oben beschrieben als Katalysator eingesetzt werden, wobei sie vorgelegt oder aber als Feststoff oder Lösung zu den Edukten und der Base zugegeben werden.

In einer vorteilhaften Ausgestaltung wird neben dem festen Palladiumkomplex zusätzlich noch der entsprechende Ligand, also der im festen Palladiumkomplex verwendete Bis-(Dialkylphosphinoferrocen)-Ligand, zugegeben.

Hier hat sich eine Zugabe im Verhältnis von festem Palladiumkomplex zu Bis-(Dialkylphosphinoferrocen)-Ligand im Bereich von 1 zu 10 bis 10 zu 1, oder von 1:5 bis 5:1, insbesondere von 2,5:1 bis 1: 2,5, wie zum Beispiel 2 zu 1, als sinnvoll herausgestellt. Das Verhältnis bezieht sich auf die molaren Mengen von festem Palladiumkomplex und Bis-(Dialkylphosphinoferrocen)-Ligand. Dies bedeutet zum Beispiel, dass bei Verwendung von 0,2 mol% festem Komplex 0,1 mol% Bis-(Dialkylphosphinoferrocen)-Ligand zugegeben werden. Der feste Palladiumkomplex kann aber auch mit zusätzlichem Bis-(Dialkylphosphinoferrocen)-Liganden im festen Zustand gemischt und so eine Pulvermischung erhalten werden. Diese Pulvermischung ist lagerstabil und kann einfach gehandhabt werden.

### Beispiele

### Beispiel 1: Herstellung der Katalysatorlösung

1,1'-Bis(diisopropylphosphino)ferrocen (dippf) (213 mg, 0.50 mmol) wurde in Diethylether (5 mL) gelöst und 1,3-Divinyl-1,1,3,3-tetrametyhldisiloxanpalladium(0) (Pd-VTS) in 2,4,6,8-Tetramethylcyclotetrasiloxan (0.50 mL, 0.50 mmol Pd) zugetropft. Das orange Gemisch wurde 1 Stunde bei Raumtemperatur gerührt.

### Beispiel 2: Herstellung des festen Katalysators [Pd(dippf)(maleimid)]

Es wurde zunächst wie in Beispiel 1 verfahren. Anschließend wurde Maleimid (99.1 mg, 1.00 mmol) in Diethylether (5 mL) zugegeben und für 10 Minuten in einem Ultraschallbad behandelt, woraufhin sich ein gelber Niederschlag bildete. Nach dem Absetzen des Feststoffes wurde die überstehende Lösung entfernt und der Rückstand mit Diethylether gewaschen (3 × 5 mL). Durch Trocknen im Vakuum (10⁻² mbar) konnte der gewünschte Palladium-Komplex als gelber Feststoff isoliert werden (291 mg, 0.47 mmol, 94%).

Analytische Daten:
¹H NMR (400 MHz, Dioxan-ds): *δ*= 7.84 (s, 1 H, N-H), 4.44 - 4.39 (m, 6 H, Ferrocen-H), 4.37 - 4.33 (m, 2 H, Ferrocen-H), 2.53 - 2.40 (m, 2 H), 2.35 - 2.20 (m, 2 H), 1.34 (d, J=7.0 Hz, 3 H), 1.30 (d, J=7.0 Hz, 3 H), 1.26 (d, *J*=7.3 Hz, 3 H), 1.22 (d, J=7.3 Hz, 6 H), 1.18 (d, J=7.3 Hz, 3 H), 1.13 (d, J=7.3 Hz, 3 H), 1.09 ppm (d, J=7.3 Hz, 3 H).
³¹P-NMR (162 MHz, Dioxan-*d₈*): *δ*=: 38.87 ppm (s, 2 P).

### Beispiel 3: Synthesen im präparativen Maßstab

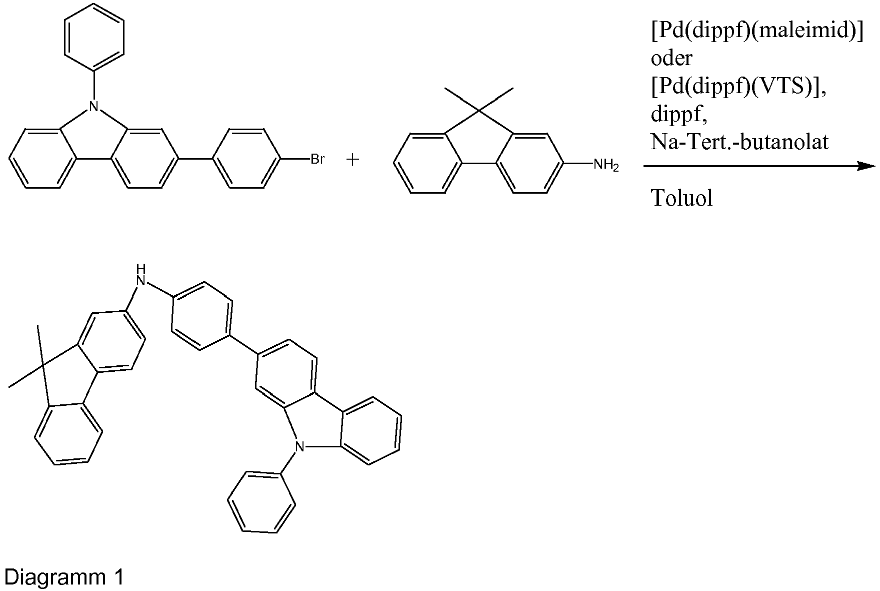

### Beispiel 3a: Verwendung von [Pd(dippf)(maleimid)] als Palladiumkomplex

In einem trockenen Dreihalskolben mit Rückflusskühler, Gaseinlass, Überdruckventil und Magnetrührstab wurden das Arylbromid 132 (7.97 g, 20.0 mmol), das Amin 131 (4.17 g, 20.0 mmol), 1,1'-Bis(diisopropylphosphino)ferrocen (8.5 mg, 0.02 mmol) und Natrium-tert-butanolat (2.35 g, 24.0 mmol) vorgelegt und durch mehrfaches Evakuieren und Begasen unter eine Stickstoffatmosphäre gebracht. Anschließend wurden Toluol (30 mL) und [Pd(dippf)(maleimid)] (24.9 mg, 0.04 mmol, aus Beispiel 2) in Toluol (10 mL) zugegeben und das Gemisch für 20 Stunden auf 70 °C erhitzt. Die Reaktion wurde durch Dünnschichtchromatographie verfolgt und festegestellt, dass nach dieser Zeit der volle Umsatz erreicht war. Nach dem Abkühlen auf Raumtemperatur wurde Wasser (60 mL) zugegeben und mit Dichlormethan (150 mL) extrahiert. Die organische Phase wurde abgetrennt, über Magnesiumsulfat (5 g) getrocknet und durch basisches Aluminiumoxid (10 g) filtriert. Das Lösungsmittel wurde im Vakuum entfernt (40 °C, 10 mbar) und der leicht gelbe Rückstand mit Diethylether (3 x 20 mL) gewaschen. Nach dem Trocknen im Vakuum (2h, 10⁻² mbar) wurde das Produkt als farbloser Feststoff erhalten (10.2 g, 19.4 mmol, 97%).
¹H NMR (400 MHz, Chloroform-*d*): *δ*= 8.41 (d, *J*=1.3 Hz, 1 H), 8.26 (d, *J*=7.8 Hz, 1 H), 7.75 - 7.60 (m, 9 H), 7.55 - 7.44 (m, 5 H), 7.41 - 7.21 (m, 6 H), 7.13 (d, *J*=7.0 Hz, 1 H), 5.91 (s, 1 H), 1.55 ppm (s, 6 H).
¹³C NMR (101 MHz, Chloroform-*d*): *δ*=155.3, 153.1, 142.6, 142.0, 141.3, 140.0, 139.3, 137.7, 134.6, 133.2, 132.5, 129.9 (2 C), 128.1, 127.4 (2 C), 127.0 (2 C), 126.9, 126.0 (2 C), 125.0, 123.9, 123.5 (2 C), 122.4, 120.8, 120.3, 120.0, 119.9, 119.1, 118.1, 116.8, 112.2, 110.0, 109.9, 46.8, 27.2 ppm (2 C).
Elementaranalyse: Berechnet für C₃₉H₃₀N₂: C 88.94, H 5.74, N 5.32; gefunden: C 88.64, H 5.91, N 5.22.

### Beispiel 3b: Verwendung von [Pd(dippf)(VTS)] als Palladiumkomplex

Für diese Reaktion wurde eine Katalysatorstammlösung ähnlich wie in Beispiel 1 beschrieben aus 1,1'-Bis(diisopropylphosphino)ferrocen (213,8 mg, 0.50 mmol), Toluol (0.5 mL) und einem Gemisch aus 1,3-Divinyl-1,1,3,3-tetrametyhldisiloxanpalladium(0) (Pd-VTS) in 2,4,6,8-Tetramethylcyclotetrasiloxan (0,5 mL, 10.87% Palladium) hergestellt, verwendet. Das Gemisch wurde eine Stunde bei Raumtemperatur gerührt.
In einem trockenen Dreihalskolben mit Rückflusskühler, Gaseinlass, Überdruckventil und Magnetrührstab wurden das Arylbromid 132 (7.97 g, 20.0 mmol), das Amin 131 (4.17 g, 20.0 mmol) und Natrium-tert-butanolat (2.35 g, 24.0 mmol) vorgelegt und durch Evakuieren und Begasen unter eine Stickstoffatmosphäre gebracht. Anschließend wurden Toluol (40 mL) und die Katalysatorstammlösung (100 µL, 91.8 mg, 0.04 mmol Palladium) zugegeben und das Gemisch für 20 Stunden auf 70 °C erhitzt. Die Reaktion wurde durch Dünnschichtchromatographie verfolgt und festgestellt, dass nach dieser Zeit der volle Umsatz erreicht war. Nach dem Abkühlen auf Raumtemperatur wurde Wasser (60 mL) zugegeben und mit Dichlormethan (150 mL) extrahiert. Die organische Phase wurde abgetrennt, über Magnesiumsulfat (5 g) getrocknet und durch basisches Aluminiumoxid (10 g) filtriert. Das Lösungsmittel wurde im Vakuum entfernt (40 °C, 10 mbar) und der leicht gelbe Rückstand mit Ether (3 × 20 mL) gewaschen. Nach dem Trocknen im Vakuum (2h, 10⁻² mbar) wurde das Produkt als farbloser Feststoff erhalten (9,71 g, 18.4 mmol, 92%).

Die Reaktionsgleichung ist in Diagramm 1 abgebildet. Eine Mehrfacharylierung des primären Amins 131 durch das Arylbromid 132 wurde in keinem der Beispiele beobachtet.

### Beispiele 4a bis 4i:

### Allgemeine Vorschrift:

Alle Versuche wurden in 20 mL Headspace-Vials für die Gaschromatographie durchgeführt, die mit Aluminium-Bördelkappen mit Teflon-beschichteten Butylgummi-Septen verschlossen wurden (beides erhältlich z.B. bei der Firma *VWR*). Zur Temperierung der Gefäße wurden 8 cm hohe zylindrische Aluminiumblöcke verwendet, die in ihrem Durchmesser genau dem der Heizplatten von Labor-Magnetrührern (z.B. Heidolph Mr 2002) entsprechen. Diese Aluminiumblöcke wurden mit zehn 7 cm tiefen Bohrungen vom Durchmesser der Reaktionsgefäße und einer Bohrung zur Aufnahme eines Temperaturfühlers versehen.

Zum gleichzeitigen Evakuieren und Rückfüllen von jeweils zehn Gefäßen wurden Vakuumverteiler zum Anschluss an die Schlenk-Linie angefertigt. Dazu wurden jeweils zehn vakuumfeste 3 mm Teflonschläuche jeweils an einem Ende mit Adaptern zur Aufnahme von Luer-Lock-Spritzennadeln verbunden und dem anderen Ende an ein Stahlrohr angeschlossen, das über einen Vakuumschlauch mit der Schlenk-Linie verbunden werden kann.

Zur Durchführung der Versuche wurden das Arylbromid (1.00 mmol), das entsprechende primäre Amin (1.00 mmol) und Natrium-*tert*-butanolat (118 mg, 1.20 mmol) an der Luft in die Reaktionsgefäße eingewogen, 20 mm Magnet-Rührkerne zugegeben und die Gefäße mittels einer Bördelzange mit Septumkappen luftdicht verschlossen. Jeweils 10 Reaktionsgefäße wurden in die Bohrungen eines Aluminiumblocks gesteckt und über Hohlnadeln, die durch die Septenkappen gebohrt wurden, mit dem Vakuumverteiler verbunden.

Die Reaktionsgefäße wurden danach gemeinsam dreimal hintereinander evakuiert und mit Stickstoff begast. Nachdem die Reaktionsgefäße auf diese Weise mit einer Inertgasatmosphäre versehen waren, wurde an der Vakuumlinie über ein Überdruckventil ein Druckausgleich mit der Außenatmosphäre hergestellt. Mit Hilfe einer Spritze wurde eine Stammlösung von [Pd(dippf)(maleimid)] (1.24 mg, 0.002 mmol) und dippf (0.85 mg, 0.002 mmol) in Toluol (2 mL) durch die Septenkappen hindurch injiziert. Danach wurde der Aluminiumblock auf 70 °C gebracht und die Nadeln des Vakuumverteilers entfernt.
Nach 20 h Reaktionszeit wurden die Gefäße nach dem Abkühlen vorsichtig geöffnet und das Reaktionsmedium mit Dichlormethan (30 mL) und Wasser (30 mL) verdünnt. Die wässrige Phase wurde mit 1N Salzsäure auf pH = 7 gestellt, von der organischen Phase getrennt und mit Dichlormethan (2 × 20 mL) extrahiert. Die vereinigten organischen Phasen wurden über Magnesiumsulfat getrocknet, filtriert und schließlich Dünnschichtchromatographisch untersucht.
Das Lösungsmittel wurde im Vakuum entfernt (40°C, 500 mbar) und das verbliebene Rohprodukt säulenchromatographisch (basisches Al₂O₃, Diethylether: Hexan oder Ethylacetat: Hexan) gereinigt.

### Beispiel 4a: Synthese von Verbindung 3a:

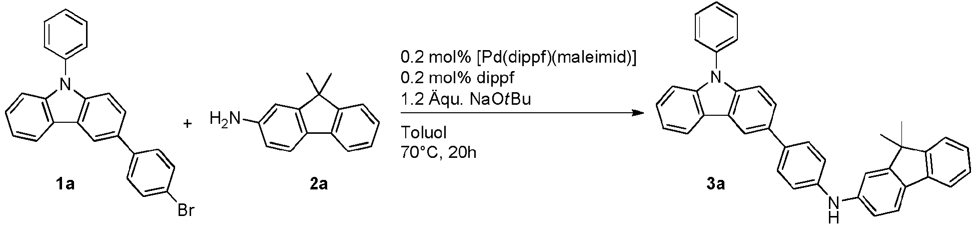

Nach der allgemeinen Vorschrift wurde Verbindung **3a** ausgehend von **1a** (398 mg, 1.00 mmol) und **2a** (209 mg, 1.00 mmol) dargestellt und konnte säulenchromatographisch (Al₂O₃, Diethylether: Hexan = 1:1) in 94% Ausbeute (496 mg, 0.94 mmol) isoliert werden. ¹H NMR (400 MHz, Chloroform-*d*): *δ* = 8.41 (d, *J*=1.3 Hz, 1 H), 8.26 (d, *J*=7.8 Hz, 1 H), 7.75 - 7.60 (m, 9 H), 7.55 - 7.44 (m, 5 H), 7.41 - 7.21 (m, 6 H), 7.13 (d, *J*=7.0 Hz, 1 H), 5.91 (s, 1 H), 1.55 ppm (s, 6 H). ¹³C NMR (101 MHz, Chloroform-*d*): *δ*= 155.3, 153.1, 142.6, 142.0, 141.3, 140.0, 139.3, 137.7, 134.6, 133.2, 132.5, 129.9 (2 C), 128.1, 127.4 (2 C), 127.0 (2 C), 126.9, 126.0 (2 C), 125.0, 123.9, 123.5 (2 C), 122.4, 120.8, 120.3, 120.0, 119.9, 119.1, 118.1, 116.8, 112.2, 110.0, 109.9, 46.8, 27.2 ppm (2 C). CHN: Berechnet für C₃₉H₃₀N₂: C 88.94, H 5.74, N 5.32; gefunden: C 88.79, H 5.86, N 5.19.

### Beispiel 4b: Synthese von Verbindung 3b:

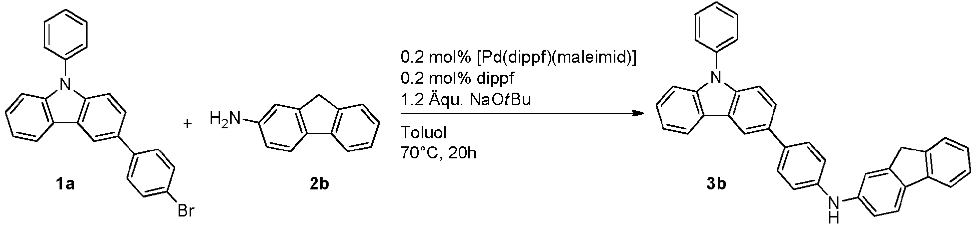

Nach der allgemeinen Vorschrift wurde Verbindung **3b** ausgehend von **1a** (398 mg, 1.00 mmol) und **2b** (181 mg, 1.00 mmol) dargestellt und konnte säulenchromatographisch (Al₂O₃, Diethylether: Hexan = 1:1) in 92% Ausbeute (460 mg, 0.92 mmol) isoliert werden. ¹H NMR (400 MHz, DMSO-*d₆*): *δ* = 8.54 (d, *J*=1.5 Hz, 1H), 8.44 (s, 1H), 8.35 (d, *J*=7.8 Hz, 1H), 7.77-□7.63 (m, 9H), 7.55 (m, 1H), 7.51 (d, *J*=7.5 Hz, 1H), 7.47 -□7.36 (m, 4H), 7.31 (m, 2H), 7.26 (d, *J*=8.8 Hz, 2H), 7.20 (dt, *J*=7.5 Hz, 1.3 Hz, 1H), 7.15 (dd, *J*=8.3 Hz, 2.0 Hz, 1H), 3.87 ppm (s, 2H). ¹³C NMR (101 MHz, DMSO-*d₆*): *δ* = 144.6, 142.7, 142.3, 142.2, 141.5, 140.5, 139.1, 136.9, 133.3, 132.6, 132.2, 130.2 (2C), 127.6, 127.5 (2C), 126.7, 126.6 (2C), 126.4, 125.3, 124.9, 124.7, 123.4, 123.0, 120.8, 120.7, 120.1, 118.8, 117.6, 117.4 (2C), 117.3, 115.9, 113.1, 110.0, 109.7 ppm. CHN: Berechnet für C₃₇H₂₆N₂: C 89.13, H 5.26, N 5.62; gefunden: C 88.83, H 5.25, N 5.54.

### Beispiel 4c: Synthese von Verbindung 3c:

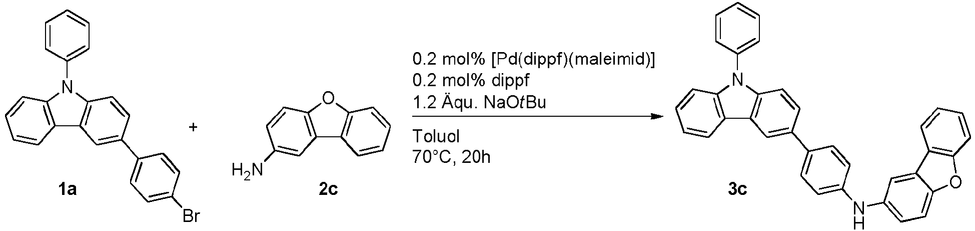

Nach der allgemeinen Vorschrift wurde Verbindung **3c** ausgehend von **1a** (398 mg, 1.00 mmol) und **2c** (183 mg, 1.00 mmol) dargestellt und konnte säulenchromatographisch (Al₂O₃, Diethylether: Hexan = 1:1) in 88% Ausbeute (442 mg, 0.88 mmol) isoliert werden. ¹H NMR (400 MHz, DMSO-*d₆*): *δ* = 8.72 (s, 1H), 8.55 (d, *J*=1.5 Hz, 1H), 8.35 (d, *J*=7.8 Hz, 1H), 7.99-7.93 (m, 2H), 7.77-7.62 (m, 7H), 7.60 (d, *J*=8.0 Hz, 1H), 7.54 (m, 1H), 7.47-7.27 (m, 9H), 7.16 ppm (dd, *J*=8.5 Hz, 1.8 Hz, 1H). ¹³C NMR (101 MHz, DMSO-*d₆*): *δ* = 157.1, 155.3, 144.0, 141.6, 140.5, 139.2, 136.9, 133.0, 132.5, 130.2 (2C), 127.6, 127.5 (2C), 126.6 (2C), 126.4, 125.6, 124.7, 124.2, 123.4, 123.0 (2C), 121.6, 120.8, 120.1, 119.7, 118.0 (2C), 117.7, 115.5, 113.1, 111.2, 110.0, 109.7, 97.9 ppm. CHN: Berechnet für C₃₆H₂₄N₂O: C 86.38, H 4.83, N 5.60; gefunden: C 86.02, H 5.04, N 5.43.

### Beispiel 4c: Synthese von Verbindung 3d:

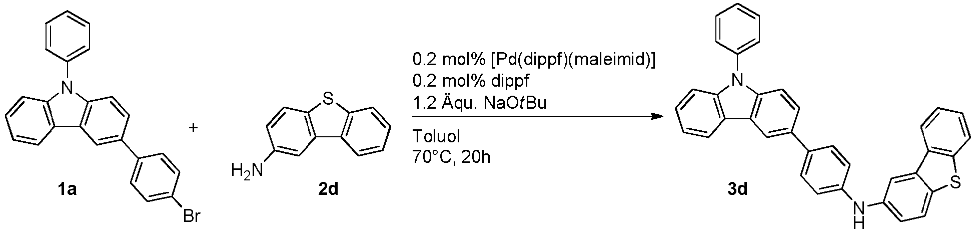

Nach der allgemeinen Vorschrift wurde Verbindung **3d** ausgehend von **1a** (398 mg, 1.00 mmol) und **2d** (199 mg, 1.00 mmol) dargestellt und konnte säulenchromatographisch (Al₂O₃, Diethylether: Hexan = 1:1) in 96% Ausbeute (496 mg, 0.96 mmol) isoliert werden. ¹H NMR (400 MHz, DMSO-*d₆*): *δ* = 8.54 (d, *J*=1.5 Hz, 1H), 8.51 (s, 1H), 8.35 (d, *J*=7.5 Hz, 1H), 8.25 (m, 1H), 8.07 (d, *J*=2.0 Hz, 1H), 7.99 (m, 1H), 7.89 (d, *J*=8.5 Hz, 1H), 7.75-7.63 (m, 7H), 7.55 (m, 1H), 7.48 (m, 2H), 7.45-7.37 (3H), 7.36-7.27 ppm (m, 4H). ¹³C NMR (101 MHz, DMSO-*d₆*): *δ* = 142.7, 141.0, 140.5, 139.5, 139.1, 136.9, 136.0, 134.9, 132.6, 132.1, 130.2 (2C), 129.7, 127.6, 127.5 (2C), 126.9, 126.6 (2C), 126.4, 124.7, 124.5, 123.6, 123.4, 123.1, 123.0, 121.9, 120.8, 120.1, 119.0, 117.6, 116.8 (2C), 110.0, 109.7, 109.2 ppm. CHN: Berechnet für C₃₆H₂₄N₂S: C 83.69, H 4.68, N 5.42, S 6.21; gefunden: C 83.40, H 4.76, N 5.35, S 6.31.

### Beispiel 4d: Synthese von Verbindung 3e:

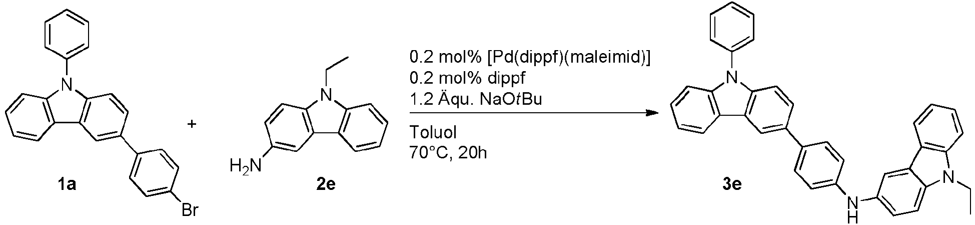

Nach der allgemeinen Vorschrift wurde Verbindung **3e** ausgehend von **1a** (398 mg, 1.00 mmol) und **2e** (221 mg, 1.00 mmol) dargestellt und konnte säulenchromatographisch (Al₂O₃, Ethylacetat: Hexan = 1:2) in 95% Ausbeute (502 mg, 0.95 mmol) isoliert werden. ¹H NMR (400 MHz, DMSO-*d₆*): *δ* = 8.50 (d, *J*=1.5 Hz, 1H), 8.33 (d, *J*=7.8 Hz, 1H), 8.14 (s, 1H), 8.11 (d, *J*=7.5 Hz, 1H), 7.96 (d, *J*=2.0 Hz, 1H), 7.72 -7.61 (m, 7H), 7.58-7.51 (m, 3H), 7.46-7.37 (m, 4H), 7.35 -7.27 (m, 2H), 7.17-7.11 (m, 3H), 4.41 (q, *J*=7.0 Hz, 2H), 1.31 ppm (t, *J*=7.0 Hz, 3H). ¹³C NMR (101 MHz, DMSO-*d₆*): *δ* = 144.6, 142.7, 142.3, 142.2, 141.5, 140.5, 139.1, 136.9, 133.3, 132.6, 132.2, 130.2 (2C), 127.6, 127.5 (2C), 126.7, 126.6 (2C), 126.4, 125.3, 124.9, 124.7, 123.4, 123.0, 120.8, 120.7, 120.1, 118.8, 117.6, 117.4 (2C), 117.3, 115.9, 113.1, 110.0, 109.7, 36.5 ppm. CHN: Berechnet für C₃₈H₂₉N₃: C 86.50, H 5.54, N 7.96; gefunden: C 86.32, H 5.63, N 7.90.

### Beispiel 4e: Synthese von Verbindung 3f:

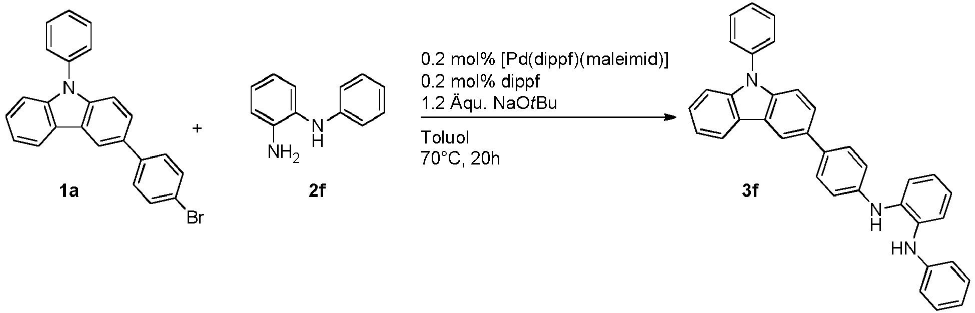

Nach der allgemeinen Vorschrift wurde Verbindung **3f** ausgehend von **1a** (398 mg, 1.00 mmol) und **2f** (188 mg, 1.00 mmol) dargestellt und konnte säulenchromatographisch (Al₂O₃, Ethylacetat: Hexan = 1:2) in 84% Ausbeute (423 mg, 0.84 mmol) isoliert werden. ¹H NMR (400 MHz, DMSO-*d₆*): *δ* = 8.49 (d, *J*=1.5 Hz, 1H), 8.33 (d, *J*=7.8 Hz, 1H), 7.72-7.61 (m, 7H), 7.53 (m, 1H), 7.46-7.37 (m, 4H), 7.36 (s, 1H), 7.34-7.26 (m, 3H), 7.19 (m, 2H), 7.10 (d, *J*=8.5 Hz, 2H), 6.99 (dd, *J*=8.7 Hz, 0.9 Hz, 2H), 6.94 (m, 2H), 6.76 ppm (m, 1H). ¹³C NMR (101 MHz, DMSO-*d₆*): *δ* = 144.5, 143.4, 140.5, 139.0, 136.9, 134.6 (2C), 132.7, 131.6, 130.2 (2C), 129.0 (2C), 127.6, 127.3 (2C), 126.6 (2C), 126.3, 124.6, 123.4, 123.0, 122.1, 122.0, 120.8, 120.1, 119.9 (2C), 119.0, 117.5, 116.7 (2C), 116.2 (2C), 109.9, 109.6 ppm. CHN: Berechnet für C₃₆H₂₇N₃: C 86.20, H 5.43, N 8.38; gefunden: C 85.82, H 5.62, N 8.22.

### Beispiel 4f: Synthese von Verbindung 3g:

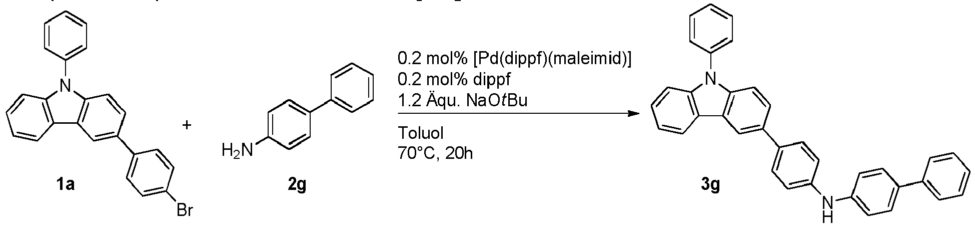

Nach der allgemeinen Vorschrift wurde Verbindung **3g** ausgehend von **1a** (398 mg, 1.00 mmol) und **2g** (169 mg, 1.00 mmol) dargestellt und konnte säulenchromatographisch (Al₂O₃, Diethylether: Hexan = 1:1) in 95% Ausbeute (462 mg, 0.95 mmol) isoliert werden. ¹H NMR (400 MHz, DMSO-*d₆*): *δ* = 8.53 (d, *J*=1.3 Hz, 1H), 8.47 (s, 1H), 8.35 (d, *J*=7.5 Hz, 1H), 7.74-7.53 (m, 12H), 7.47-7.38 (m, 5H), 7.34-7.19 ppm (m, 6H). ¹³C NMR (101 MHz, DMSO-*d₆*): *δ* = 143.0, 142.9, 141.9, 140.5, 140.1, 139.1, 136.9, 132.6, 132.5, 131.2, 130.2 (2C), 128.9 (2C), 127.6, 127.5 (2C), 127.4 (2C), 126.6 (2C), 126.4 (2C), 125.8 (2C), 124.7, 123.4, 123.0, 120.8, 120.1, 117.7, 117.5 (2C), 116.8, 110.0, 109.7 ppm. CHN: Berechnet für C₃₆H₂₆N₂: C 88.86, H 5.39, N 5.76; gefunden: C 88.49, H 5.39, N 5.68.

### Beispiel 4g: Synthese von Verbindung 3i:

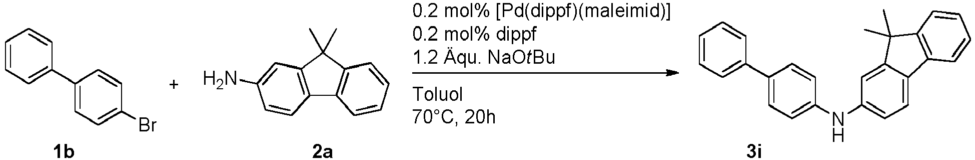

Nach der allgemeinen Vorschrift wurde Verbindung **3i** ausgehend von **1b** (259 mg, 1.00 mmol) und **2a** (209 mg, 1.00 mmol) dargestellt und konnte säulenchromatographisch (Al₂O₃, Diethylether: Hexan = 1:2) in 89% Ausbeute (323 mg, 0.89 mmol) isoliert werden. ¹H NMR (400 MHz, Chloroform-*d*): *δ* = 7.72-7.63 (m, 4H), 7.59 (m, 2H), 7.51-7.44 (m, 3H), 7.37 (m, 2H), 7.32 (t, *J*=7.0 Hz, 1H), 7.25 (br. s, 1H), 7.22 (d, *J*=8.3 Hz, 2H), 7.13 (br. d, *J*=7.5 Hz, 1H), 5.92 (br. s, 1H), 1.54 ppm (s, 6H). ¹³C NMR (101 MHz, Chloroform-d): *δ* = 155.3, 153.1, 142.7, 142.1, 140.8, 139.2, 133.5, 132.8, 128.7 (2C), 128.0 (2C), 126.9, 126.6, 126.5 (2C), 126.1, 122.4, 120.8, 119.1, 117.6 (2C), 117.2, 112.6, 46.8, 27.2 ppm (2C). CHN: Berechnet für C₂₇H₂₃N: C 89.71, H 6.41, N 3.87; gefunden: C 89.69, H 6.27, N 3.84.

### Beispiel 4h: Synthese von Verbindung 3j:

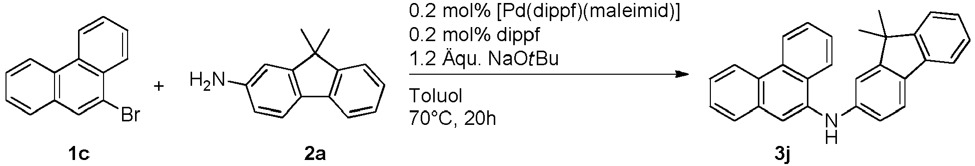

Nach der allgemeinen Vorschrift wurde Verbindung **3j** ausgehend von **1c** (268 mg, 1.00 mmol) und **2a** (209 mg, 1.00 mmol) dargestellt und konnte säulenchromatographisch (Al₂O₃, Diethylether: Hexan = 1:2) in 87% Ausbeute (335 mg, 0.87 mmol) isoliert werden. ¹H NMR (400 MHz, DMSO-*d₆*): *δ* = 8.86 (m, 1H), 8.72 (m, 1H), 8.42 (s, 1H), 8.37 (dd, *J*=8.2 Hz, 1.1 Hz, 1H), 7.77-7.65 (m, 5H), 7.63 (s, 1H), 7.51 (m, 2H), 7.45 (d, *J*=7.5 Hz, 1H), 7.32 (d, *J*=2.0 Hz, 1H), 7.27 (dt, *J*=7.5 Hz, 1.1 Hz, 1H), 7.20 (dt, *J*=7.3 Hz, 1.0 Hz, 1H), 7.13 (dd, *J*=8.3 Hz, 2.0 Hz, 1H), 1.40 ppm (s, 6H). ¹³C NMR (101 MHz, DMSO-*d₆*): *δ* = 154.7, 152.7, 144.6, 139.0, 137.8, 132.5, 131.0, 130.8, 127.5, 127.1, 127.1, 127.0, 126.9, 126.6, 126.4, 125.8, 124.5, 123.3, 123.3, 122.6, 122.5, 120.9, 118.9, 116.8, 112.4, 111.7, 46.3, 27.1 ppm (2C). CHN: Berechnet für C₂₉H₂₃N: C 90.35, H 6.01, N 3.63; gefunden: C 89.98, H 6.31, N 3.49.

### Beispiel 4i: Synthese von Verbindung 3k:

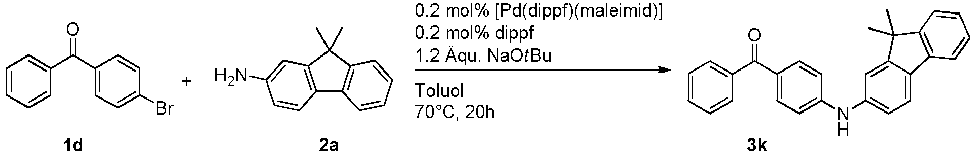

Nach der allgemeinen Vorschrift wurde Verbindung **3k** ausgehend von **1d** (269 mg, 1.00 mmol) und **2a** (209 mg, 1.00 mmol) dargestellt und konnte säulenchromatographisch (Al₂O₃, Diethylether: Hexan = 2:1) in 76% Ausbeute (296 mg, 0.76 mmol) isoliert werden. ¹H NMR (400 MHz, DMSO-*d₆*): *δ* = 9.08 (s, 1H), 7.80-7.45 (m, 10H), 7.41-7.35 (m, 1H), 7.33-7.13 (m, 5H), 1.43 ppm (s, 6H). ¹³C NMR (101 MHz, DMSO-*d₆*): *δ* = 193.6, 154.8, 152.9, 148.7, 140.6, 138.5 (2C), 132.9, 132.4 (2C), 131.5, 129.0 (2C), 128.3 (2C), 127.0, 126.7, 126.4, 122.6, 120.9, 119.3, 118.6, 114.1, 113.9 (2C), 46.4, 26.9 ppm (2C). CHN: Berechnet für C₂₈H₂₃NO: C 86.34, H 5.95, N 3.60; gefunden: C 86.05, H 6.10, N 3.52.

## Patentansprüche

1. Verfahren zur selektiven Arylierung eines primären aromatischen Amins der Formel A-NH₂ mit einer aromatischen Verbindung der Formel X-B zu einem sekundären aromatischen Amin A-NH-B, wobei die Reste A und B unabhängig voneinander gleiche oder verschiedene, substituierte oder unsubstituierte aromatische Reste sind und der Rest X ein Halogen oder ein Trifluormethylsulfonsäurerest ist, wobei in dem sekundären aromatischen Amin die aromatischen Kohlenstoffatome direkt an das Stickstoffatom gebunden sind und mindestens einer der Reste A oder B eine Biphenyleinheit aufweist und die Reaktion in Gegenwart einer Base und eines Palladiumkomplexes durchgeführt wird, wobei das Palladiumatom von mindestens einem Bis-(Dialkylphosphinoferrocen)-Liganden komplexiert wird.

2. Verfahren nach Anspruch 1, wobei die Alkylsubstituenten der Bis-(Dialkylphosphinoferrocen)-Liganden zwei bis fünf Kohlenstoffatome aufweisen.

3. Verfahren nach Anspruch 1 oder 2, wobei die Alkylsubstituenten ausgewählt sind aus der Gruppe bestehend aus Isopropyl, Isobutyl, tert.-Butyl und deren Kombinationen.

4. Verfahren nach einem der Ansprüche 1 bis 3, wobei beide aromatische Substituenten A und B eine Biphenyleinheit aufweisen, welche gleich oder voneinander verschieden sind.

5. Verfahren nach einem der Ansprüche 1 bis 4, wobei die Biphenyleinheit direkt an das sekundäre Stickstoffatom des Amins gebunden ist.

6. Verfahren nach einem der vorstehenden Ansprüche, wobei die Biphenyleinheit eine verbrückte Biphenyleinheit der Formel 2 oder 3 ist 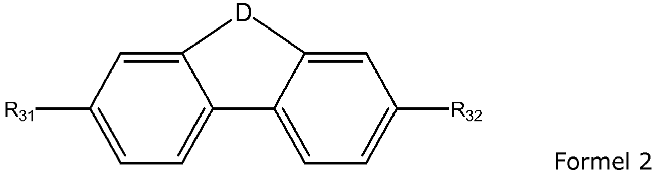 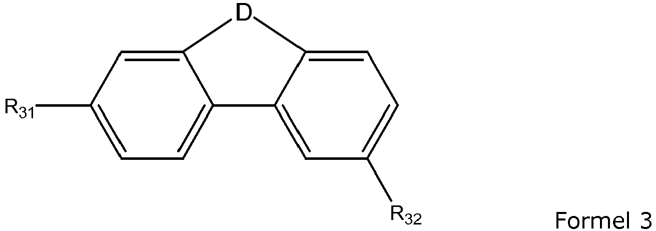 wobei D Sauerstoff, Schwefel, Stickstoff oder Kohlenstoff sein und im Fall von Stickstoff einfach oder im Fall von Kohlenstoff zweifach mit Methyl, Ethyl, Biphenyl, Naphthyl oder Phenyl substituiert sein kann.

7. Verfahren nach einem oder mehreren der vorstehenden Ansprüche, wobei die Biphenyleinheit 2-Fluoren, 3-Fluoren, 2-(9,9-Diphenylfluoren), 2-(9,9-Dimethylfluoren), 3-(9,9-Diphenylfluoren), 3-(9,9-Dimethylfluoren), 3-(4-phenyl)-9-phenyl-9H-carbazol, 3-(4-phenyl)-9-methyl-9H-carbazol, 3-(4-phenyl)-9-Biphenyl-9H-carbazol, 2-(4-phenyl)-9-phenyl-9H-carbazol, 2-(4-phenyl)-9-methyl-9H-carbazol, 2-(4-phenyl)-9-Biphenyl-9H-carbazol, 3-(4-Biphenyl)-9-phenyl-9H-carbazol, 3-(4-Biphenyl)-9-methyl-9H-carbazol, 3-(4-Biphenyl)-9-Biphenyl-9H-carbazol, 2-(4-Biphenyl)-9-phenyl-9H-carbazol, 2-(4-Biphenyl)-9-methyl-9H-carbazol, 2-(4-Biphenyl)-9-Biphenyl-9H-carbazol, -- 3-(9-phenyl-9H-carbazol), 3-(9-methyl-9H-carbazol), 3-(9-Biphenyl-9H-carbazol), 2-(9-phenyl-9H-carbazol), 2-(9-methyl-9H-carbazol), 2-(9-Biphenyl-9H-carbazol) oder Triphenylene ist

8. Verfahren nach einem oder mehreren der vorstehenden Ansprüche, wobei der Palladiumkomplex in Mengen von 0,01 mol% bis 1,5 mol%, bezogen auf die molare Gesamtmenge an beiden Edukten, dem primären aromatischen Amin A-NH₂ und dem Halogenaromaten X-B, eingesetzt wird.

9. Verfahren nach einem oder mehreren der vorstehenden Ansprüche, wobei der Palladiumkomplex als Feststoff, Lösung oder Pulvermischung mit einem festen Bis-(Dialkylphosphinoferrocen) verwendet wird.

10. Verfahren nach einem der vorstehenden Ansprüche enthaltend die Schritte:
- Bereitstellen des primären aromatischen Amins A-NH₂, des Halogenaromaten X-B, eines geeigneten Lösemittels und gegebenenfalls eines Bis-(Dialkylphosphinoferrocens) in einem Reaktionsgefäß;
- Zugeben eines Palladiumkomplexes, bei welchem das Palladiumatom von mindestens einem Bis-(Dialkylphosphinoferrocen)-Liganden komplexiert wird, in Form eines Feststoffs oder einer Lösung;
- Erwärmen des so erhaltenen Reaktionsgemisches in dem Reaktionsgefäß;
- Abtrennen des Reaktionsproduktes, eines sekundären aromatischen Amin A-NH-B; und
- Gegebenenfalls Reinigung des sekundären aromatischen Amin A-NH-B.

11. Verfahren nach einem oder mehreren der vorstehenden Ansprüche, wobei die Verbindungen 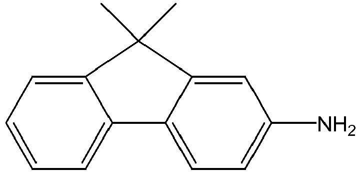 und 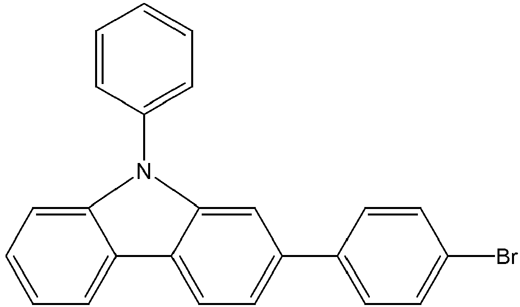 vorhanden sind.

12. Verfahren nach einem oder mehreren der vorstehenden Ansprüche, wobei in dem Palladiumkomplex das Palladiumatom zusätzlich von 2,4,6,8-Tetramethylcyclotetrasiloxan, Bis(dibenzylidenaceton) oder Maleimid komplexiert wird.

13. Verfahren nach einem oder mehreren der vorstehenden Ansprüche, wobei das Palladiumatom in dem Palladiumkomplex von 1,1'-Bis(diisopropylphosphino)ferrocen komplexiert wird.

14. Die chemische Verbindung 1,1'-Bis(diisopropylphosphino)ferrocenpalladium(0)-maleimid [Pd(dippf)(maleimid)]; 1,1'-Bis(diisopropylphosphino)ferrocenpalladium(0)-1,3-Divinyl-1,1,3,3,-tetramethyldisiloxan[Pd(dippf)(VTS)]; 1,1'-Bis(diisopropylphosphino)ferrocenpalladium(0)-bis(dibenzylidenaceton)[Pd(dippf)(dba)]; 1,1'-Bis(diisopropylphosphino)ferrocenpalladium(0)- naphthochinon [Pd(dippf)(Naphthochinon)]; 1,1'-Bis(diisopropylphosphino)ferrocenpalladium(0)-maleinsäureanhydrid [Pd(dippf)(Maleinsäureanhydrid)] oder 1,1'-Bis(diisopropylphosphino)ferrocenpalladium(0)-maleinsäurediethylester[Pd(dippf)(Maleinsäurediethylester)].

15. Verwendung einer Verbindung 1,1'-Bis(diisopropylphosphino)ferrocenpalladium(0)-maleimid [Pd(dippf)(maleimid)]; 1,1'-Bis(diisopropylphosphino)ferrocenpalladium(0)-1,3-Divinyl-1,1,3,3,-tetramethyldisiloxan[Pd(dippf)(VTS)]; 1,1'-Bis(diisopropylphosphino)ferrocenpalladium(0)-bis(dibenzylidenaceton) [Pd(dippf)(dba)]; 1,1'-Bis(diisopropylphosphino)ferrocenpalladium(0)- naphthochinon [Pd(dippf)(Naphthochinon)]; 1,1'-Bis(diisopropylphosphino)ferrocenpalladium(0)-maleinsäureanhydrid [Pd(dippf)(Maleinsäureanhydrid)]; 1,1'-Bis(diisopropylphosphino)ferrocenpalladium(0)-maleinsäurediethylester [Pd(dippf)(Maleinsäurediethylester)] oder 1,1'-Bis(diisopropylphosphino)ferrocenpalladium(0)-Norbornen [Pd(dippf)(Norbornen)] als Katalysator für die Hartwig-Buchwald-Kupplung.

## Claims

1. Method for selective arylation of a primary aromatic amine of the formula A-NH₂ with an aromatic compound of the formula X-B to a secondary aromatic amine A-NH-B, wherein the radicals A and B, independently of one another, are the same or different, substituted or unsubstituted aromatic radicals, and X is a halogen or a trifluoromethyl sulfonic acid rest, wherein, in the secondary aromatic amine, the aromatic carbon atom is directly bound to the nitrogen atom, and at least one of the rests A or B has a biphenyl unit, and the reaction is carried out in the presence of a base and palladium complex, wherein the palladium atom is complexed by at least one bis-(dialkylphosphinoferrocene)-ligand.

2. Method according to claim 1, wherein the alkyl substituents of the bis-(dialkylphosphinoferrocene) ligands have two to five carbon atoms.

3. Method according to claim 1 or 2, wherein the alkyl substituents are selected from the group consisting of isopropyl, isobutyl, tert.-butyl, and combinations thereof.

4. Method according to one of claims 1 through 3, wherein both of the aromatic substituents A and B have a biphenyl unit which are the same or different from each other.

5. Method according to one of claims 1 through 4, wherein the biphenyl unit is bound directly to the secondary nitrogen atom of the amine.

6. Method according to one of the previous claims, wherein the biphenyl unit is a bridged biphenyl unit of formula 2 or 3 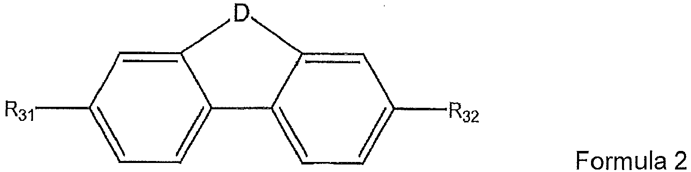 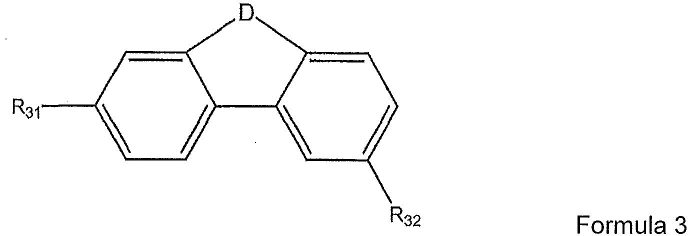 wherein D can be oxygen, sulfur, nitrogen, or carbon and may be substituted once (in the case of nitrogen) or twice (in the case of carbon) with methyl, ethyl, biphenyl, naphthyl, or phenyl.

7. Method according to one or more of the previous claims, wherein the biphenyl unit is 2-fluorene, 3-fluorene, 2-(9,9-diphenylfluorene), 2-(9,9-dimethylfluorene), 3-(9,9-diphenylfluorene), 3-(9,9-dimethylfluorene), 3-(4-phenyl)-9-phenyl-9H-carbazole, 3-(4-phenyl)-9-methyl-9H-carbazole, 3-(4-phenyl)-9-biphenyl-9H-carbazole, 2-(4-phenyl)-9-phenyl-9H-carbazole, 2-(4-phenyl)-9-methyl-9H-carbazole, 2-(4-phenyl)-9-biphenyl-9H-carbazole, 3-(4-biphenyl)-9-phenyl-9H-carbazole, 3-(4-biphenyl)-9-methyl-9H-carbazole, 3-(4-biphenyl)-9-biphenyl-9H-carbazole, 2-(4-biphenyl)-9-phenyl-9H-carbazole, 2-(4-biphenyl)-9-methyl-9H-carbazole, 2-(4-biphenyl)-9-biphenyl-9H-carbazole, -- 3-(9-phenyl-9H-carbazole), 3-(9-methyl-9H-carbazole), 3-(9-biphenyl-9H-carbazole), 2-(9-phenyl-9H-carbazole), 2-(9-methyl-9H-carbazole), 2-(9-biphenyl-9H-carbazole), or triphenylene.

8. Method according to one or more of the previous claims, wherein the palladium complex is used in quantities of 0.01 mol% to 1.5 mol%, in relation to the total molar quantity of both educts, the primary aromatic amine A-NH₂, and the halogen aromatic X-B.

9. Method according to one or more of the previous claims, wherein the palladium complex is used as a solid, solution, or powder mixture with a solid bis-(dialkylphosphinoferrocene).

10. Method according to one of the previous claims, containing the steps:
- Providing the primary aromatic amine A-NH₂, the halogen aromatic X-B, a suitable solvent, and, where applicable, a bis-(dialkylphosphinoferrocene) in a reaction vessel;
- Adding a palladium complex, in which the palladium atom is complexed by at least one bis-(dialkylphosphinoferrocene) ligand, in the form of a solid or a solution;
- Heating the reaction mixture thus obtained in the reaction vessel;
- Separating the reaction product, a secondary aromatic amine A-NH-B; and,
- where applicable, purifying the secondary aromatic amine A-NH-B.

11. Method according to one or more of the previous claims, wherein the compounds, 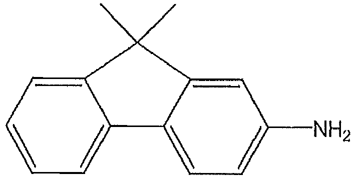 and 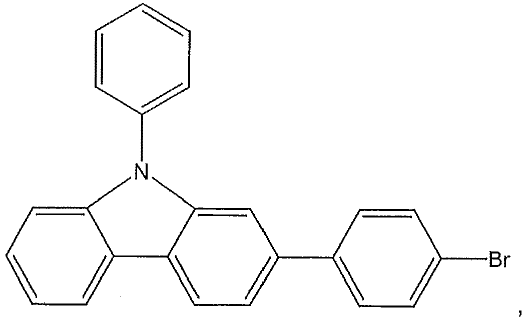 are present.

12. Method according to one or more of the previous claims, wherein, in the palladium complex, the palladium atom is additionally complexed by 2,4,6,8-tetramethylcyclotetrasiloxane, bis(dibenzylideneacetone), or maleimide.

13. Method according to one or more of the previous claims, wherein the palladium atom in the palladium complex is complexed by 1,1'-bis(diisopropylphosphino)ferrocene.

14. The chemical compounds, 1,1'-bis(diisopropylphosphino)ferrocene palladium(0)-maleimide[Pd(dippf)(maleimide)]; 1,1'-bis(diisopropylphosphino)ferrocene palladium(0)-1,3-divinyl-1,1,3,3,-tetramethyldisiloxane [Pd(dippf)(VTS)]; 1,1'-bis(diisopropylphosphino)ferrocene palladium(0)-bis(dibenzylideneacetone) [Pd(dippf)(dba)]; 1,1'-bis(diisopropylphosphino)ferrocene palladium(0)-naphthoquinone [Pd(dippf)(naphthoquinone)]; 1,1'-bis(diisopropylphosphino)ferrocene palladium(0)-maleic acid anhydride [Pd(dippf)(maleic acid anhydride)]; or 1,1'-bis(diisopropylphosphino)ferrocene palladium(0)-maleic acid diethyl ester [Pd(dippf)(maleic acid diethyl ester)].

15. Use of a compound, 1,1'-bis(diisopropylphosphino)ferrocene palladium(0)-maleimide [Pd(dippf)(maleimide)]; 1,1'-bis(diisopropylphosphino)ferrocene palladium(0)-1,3-divinyl-1,1,3,3,-tetramethyldisiloxane [Pd(dippf)(VTS)]; 1,1'-bis(diisopropylphosphino)ferrocene palladium(0)-bis(dibenzylideneacetone) [Pd(dippf)(dba)]; 1,1'-bis(diisopropylphosphino)ferrocene palladium(0)-naphthoquinone [Pd(dippf)(naphthoquinone)]; 1,1'-bis(diisopropylphosphino)ferrocene palladium(0)-maleic acid anhydride [Pd(dippf)(maleic acid anhydride)]; 1,1'-bis(diisopropylphosphino)ferrocene palladium(0)-maleic acid diethyl ester [Pd(dippf)(maleic acid diethyl ester)]; or 1,1'-bis(diisopropylphosphino)ferrocene palladium(0)-norbornene [Pd(dippf)(norbornene)], as catalyst for the Hartwig-Buchwald coupling.

## Revendications

1. Procédé pour l'arylation sélective d'une amine primaire aromatique de formule A-NH₂ avec un composé aromatique de formule X-B en une amine secondaire aromatique A-NH-B, les radicaux A et B représentant, indépendamment l'un de l'autre, des radicaux aromatiques identiques ou différents, substitués ou non substitués et le radical X représentant un halogène ou un radical acide trifluorométhylsulfonique, les atomes de carbone aromatiques dans l'amine secondaire aromatique étant liés directement à l'atome d'azote et au moins un des radicaux A ou B présentant une unité biphényle et la réaction étant réalisée en présence d'une base et d'un complexe du palladium, l'atome de palladium étant complexé par au moins un ligand de type bis-(dialkylphosphinoferrocène).

2. Procédé selon la revendication 1, les substituants alkyle des ligands de type bis-(dialkylphosphinoferrocène) présentant deux à cinq atomes de carbone.

3. Procédé selon la revendication 1 ou 2, les substituants alkyle étant choisis dans le groupe constitué par isopropyle, isobutyle, tert-butyle et leurs combinaisons.

4. Procédé selon l'une quelconque des revendications 1 à 3, les deux substituants aromatiques A et B présentant une unité biphényle, l'une étant identique à l'autre ou différente de l'autre.

5. Procédé selon l'une quelconque des revendications 1 à 4, l'unité biphényle étant liée directement à l'atome d'azote secondaire de l'amine.

6. Procédé selon l'une quelconque des revendications précédentes, l'unité biphényle étant une unité biphényle pontée de formule 2 ou de formule 3 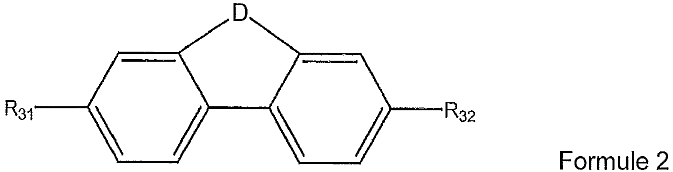 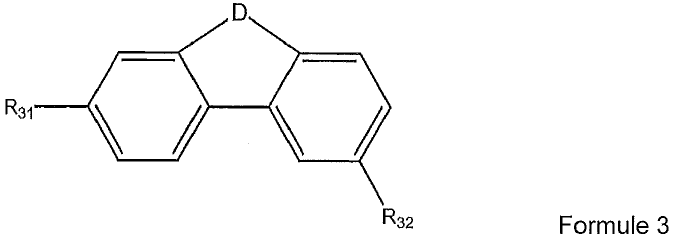 D représentant oxygène, soufre, azote ou carbone et, dans le cas de l'azote, pouvant être monosubstitué ou, dans le cas du carbone, pouvant être disubstitué par méthyle, éthyle, biphényle, naphtyle ou phényle.

7. Procédé selon l'une ou plusieurs des revendications précédentes, l'unité biphényle étant le 2-fluorène, le 3-fluorène, le 2-(9,9-diphénylfluorène), le 2-(9,9-diméthylfluorène), le 3-(9,9-diphénylfluorène), le 3-(9,9-diméthylfluorène), le 3-(4-phényl)-9-phényl-9H-carbazole, le 3-(4-phényl)-9-méthyl-9H-carbazole, le 3-(4-phényl)-9-biphényl-9H-carbazole, le 2-(4-phényl)-9-phényl-9H-carbazole, le 2-(4-phényl)-9-méthyl-9H-carbazole, le 2-(4-phényl)-9-biphényl-9H-carbazole, le 3-(4-biphényl)-9-phényl-9H-carbazole, le 3-(4-biphényl)-9-méthyl-9H-carbazole, le 3-(4-biphényl)-9-biphényl-9H-carbazole, le 2-(4-biphényl)-9-phényl-9H-carbazole, le 2-(4-biphényl)-9-méthyl-9H-carbazole, le 2-(4-biphényl)-9-biphényl-9H-carbazole, le 3-(9-phényl-9H-carbazole), le 3-(9-méthyl-9H-carbazole), le 3-(9-biphényl-9H-carbazole), le 2-(9-phényl-9H-carbazole), le 2-(9-méthyl-9H-carbazole), le 2-(9-biphényl-9H-carbazole) ou le triphénylène.

8. Procédé selon l'une ou plusieurs des revendications précédentes, le complexe de palladium étant utilisé en des quantités de 0,01 % en mole à 1,5 % en mole, par rapport à la quantité molaire totale des deux produits de départ, l'amine primaire aromatique A-NH₂ et l'halogénoaromatique X-B.

9. Procédé selon l'une ou plusieurs des revendications précédentes, le complexe de palladium étant utilisé sous forme de solide, de solution ou de mélange de poudres avec un bis-(dialkylphosphinoferrocène) solide.

10. Procédé selon l'une ou plusieurs des revendications précédentes, contenant les étapes :
- préparation de l'amine primaire aromatique A-NH₂, de l'halogénoaromatique X-B, d'un solvant approprié et le cas échéant d'un bis-(dialkylphosphinoferrocène) dans un récipient de réaction ;
- addition d'un complexe de palladium dans lequel l'atome de palladium est complexé par au moins un ligand de type bis-(dialkylphosphinoferrocène), sous forme d'un solide ou d'une solution ;
- chauffage du mélange réactionnel ainsi obtenu dans le récipient de réaction ;
- séparation du produit de réaction, d'une amine secondaire aromatique A-NH-B ; et
- le cas échéant purification de l'amine secondaire aromatique A-NH-B.

11. Procédé selon l'une ou plusieurs des revendications précédentes, les composés 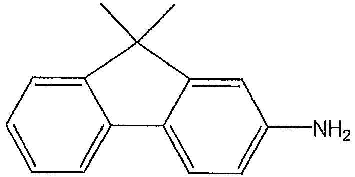 et 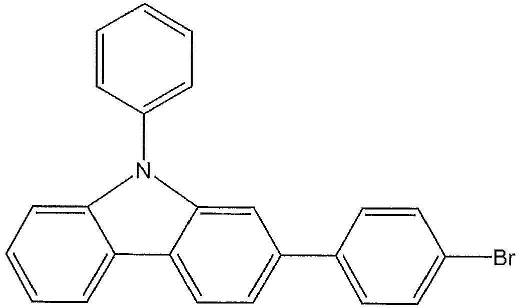 étant présents.

12. Procédé selon l'une ou plusieurs des revendications précédentes, l'atome de palladium dans le complexe de palladium étant en outre complexé par le 2,4,6,8-tétraméthylcyclotétrasiloxane, la bis(dibenzylidénacétone) ou le maléimide.

13. Procédé selon l'une ou plusieurs des revendications précédentes, l'atome de palladium dans le complexe de palladium étant complexé par le 1,1'-bis(diisopropylphosphino)ferrocène.

14. Composé chimique de type 1,1'-bis(diisopropylphosphino)ferrocène-palladium(0)-maléimide [Pd(dippf)(maléimide)] ; 1,1'-bis(diisopropylphosphino)ferrocène-palladium(0)-1,3-divinyl-1,1,3,3,-tétraméthyldisiloxane [Pd(dippf)(VTS)] ; 1,1'-bis(diisopropylphosphino)ferrocène-palladium(0)-bis(dibenzylidénacétone) [Pd(dippf)(dba)] ; 1,1'-bis(diisopropylphosphino)ferrocène-palladium(0)-naphtoquinone [Pd(dippf)(naphtoquinone)]; 1,1'-bis(diisopropylphosphino)ferrocène-palladium(0)-anhydride d'acide maléique [Pd(dippf)(anhydride d'acide maléique)] ; ou 1,1'-bis(diisopropylphosphino)ferrocène-palladium(0)-ester diéthylique de l'acide maléique [Pd(dippf)(ester diéthylique de l'acide maléique)].

15. Utilisation d'un composé de type 1,1'-bis(diisopropylphosphino)ferrocène-palladium(0)-maléimide[Pd(dippf)(maléimide)] ; 1,1'-bis(diisopropylphosphino)ferrocéne-palladium(0)-1,3-divinyl-1,1,3,3,-tétraméthyldisiloxane [Pd(dippf)(VTS)] ; 1,1'-bis(diisopropylphosphino)ferrocène-palladium(0)-bis(dibenzylidénacétone)[Pd(dippf)(dba)]; 1,1'-bis(diisopropylphosphino)ferrocéne-palladium(0)-naphtoquinone [Pd(dippf)(naphtoquinone)] ; 1,1'-bis(diisopropylphosphino)ferrocène-palladium(0)-anhydride d'acide maléique [Pd(dippf)(anhydride d'acide maléique)] ; 1,1'-bis(diisopropylphosphino)ferrocéne-palladium(0)-ester diéthylique de l'acide maléique [Pd(dippf)(ester diéthylique de l'acide maléique)] ; ou 1,1'-bis(diisopropylphosphino)ferrocène-palladium(0)-norbornène [Pd(dippf)(norbornéne)] comme catalyseur pour le couplage de Hartwig-Buchwald.
